(19) Europäisches Patentamt
European Patent Office
Office européen des brevets

(11) **EP 3 914 893 B1**

(12) **FASCICULE DE BREVET EUROPEEN**

(45) Date de publication et mention de la délivrance du brevet:
**14.08.2024   Bulletin 2024/33**

(21) Numéro de dépôt: **20700949.9**

(22) Date de dépôt: **23.01.2020**

(51) Classification Internationale des Brevets (IPC):
**G01N 1/38** *(2006.01)*      **G01N 15/06** *(2024.01)*
**G01N 15/14** *(2024.01)*      **G01N 1/28** *(2006.01)*
**G01N 1/30** *(2006.01)*

(52) Classification Coopérative des Brevets (CPC):
**G01N 1/38; G01N 15/06; G01N 15/1468;**
G01N 1/2813; G01N 1/30; G01N 15/01;
G01N 15/12; G01N 15/1484; G01N 2015/016;
G01N 2015/1006; G01N 2015/1486

(86) Numéro de dépôt international:
**PCT/EP2020/051646**

(87) Numéro de publication internationale:
**WO 2020/152277 (30.07.2020 Gazette 2020/31)**

(54) **PROCÉDÉ ET SYSTÈME D'OPTIMISATION DE LA CONCENTRATION EN ÉLÉMENTS D'INTÉRÊT À DES FINS DE MESURES VISUELLES SUR UN ÉCHANTILLON BIOLOGIQUE**

VERFAHREN UND SYSTEM ZUR OPTIMIERUNG DER KONZENTRATION VON INTERESSIERENDEN ELEMENTEN FÜR VISUELLE MESSUNGEN AN EINER BIOLOGISCHEN PROBE

METHOD AND SYSTEM FOR OPTIMISING THE CONCENTRATION OF ELEMENTS OF INTEREST FOR VISUAL MEASUREMENTS ON A BIOLOGICAL SAMPLE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.01.2019   FR 1900590**

(43) Date de publication de la demande:
**01.12.2021   Bulletin 2021/48**

(73) Titulaire: **Erba Diagnostics Limited
Leopardstown Dublin, 18 (IE)**

(72) Inventeurs:
• **VAZIRANI, Nikhil
  Mumbai MAHARASHTRA 400049 (IN)**
• **ANDLAUER, Sylvain
  34920 LE CRES (FR)**
• **NOEL, Samantha
  34000 MONTPELLIER (FR)**

(74) Mandataire: **Regimbeau
  20, rue de Chazelles
  75847 Paris Cedex 17 (FR)**

(56) Documents cités:
**WO-A1-2015/001553      WO-A2-2006/103334**

• **NATALIE MAYHEAD ET AL: "How to "stick" leukocytes to a microscope slide?", RESEARCHGATE, 7 July 2013 (2013-07-07), XP055683422, Retrieved from the Internet <URL:https://www.researchgate.net/post/How_to_stick_leukocytes_to_a_microscope_slide2> [retrieved on 20200406]**
• **MIKROMETASTASELAB ET AL: "CYTOSPIN PREPARATION", 1 January 2006 (2006-01-01), XP055683190, Retrieved from the Internet <URL:https://ki.projectcoordinator.net/main.php/Cytospin%20jan%202006.pdf?fileitem=2785415> [retrieved on 20200406]**
• **MARTINAZZI MASSIMO: "A Slide Centrifugation Technic for Concentrating Blood Leukocytes and Nucleated Cells from Bone Marrow Bloo", AMERICAN JOURNAL OF CLINICAL PATHOLOGY, 1 December 1971 (1971-12-01), pages 719 - 722, XP009519771, ISSN: 1943-7722, DOI: 10.1093/ajcp/56.6.719**

# Description

## DOMAINE DE L'INVENTION

L'invention concerne l'analyse d'un fluide corporel pour compter et identifier des éléments d'intérêt, notamment en hématologie pour le comptage de cellules et la différenciation des cellules leucocytes et des érythrocytes. L'invention se rapporte à un procédé amélioré de prise de vue d'un échantillon de fluide corporel, ainsi qu'à un procédé et à un produit programme d'ordinateur associés.

## ETAT DE LA TECHNIQUE

De nombreuses pathologies du corps humain ou animal correspondent à une quantité anormale de leucocytes ou d'érythrocytes dans le sang, ou à une répartition anormale des leucocytes entre les cinq sous-groupes connus : lymphocytes, monocytes, basophiles, éosinophiles et neutrophiles. Par exemple, une concentration élevée en lymphocytes dans un échantillon sanguin peut être corrélée à une réponse immunitaire.

Les divers sous-groupes de cellules sanguines se distinguent en particulier par leur taille moyenne, par leur complexité membranaire et par le nombre de noyaux cellulaires (cellules anucléées, mononucléées, polynucléées). Des résultats très fiables de différenciation des éléments d'intérêt peuvent être obtenus par une analyse visuelle humaine. On cherche cependant à limiter au maximum l'intervention humaine et le temps et les coûts associés. De plus, la puissance statistique d'une différenciation réalisée à l'œil nu n'est pas satisfaisante pour des applications médicales.

Des résultats précis de comptage et de différenciation peuvent être obtenus par des procédés automatisés. L'automatisation est indispensable pour des applications à cadence d'analyse élevée.

La plupart des procédés automatisés connus reposent sur une mesure de type optique réalisée sur un échantillon de sang. Il est commun de réaliser des mesures de résistivité ou de diffraction optique de l'échantillon. Le document brevet FR 2 883 972 décrit (en relation aux Figures 4 et 5) un dispositif optique permettant une détection de la lumière diffractée par un échantillon biologique selon plusieurs angles et à plusieurs longueurs d'onde. Une différenciation leucocytaire est réalisée sur la base des mesures de diffraction. Le document brevet US 5 812 419 décrit (par exemple en relation à la Figure 31) une cellule de cytométrie de flux adaptée pour réaliser des mesures optiques sur un échantillon biologique.

La mesure par cytométrie de flux présente l'avantage d'une cadence élevée. Toutefois, un défaut majeur est la difficulté à vérifier les résultats d'analyse. Les signaux optiques sont traités de manière informatique et un résultat de répartition de éléments d'intérêt entre les sous-groupes est fourni à l'utilisateur, sans aucune manière intuitive pour l'utilisateur de vérifier les résultats.

Ainsi, ces systèmes de l'état de la technique fonctionnent en « boîte noire ». Plusieurs artefacts peuvent diminuer la fiabilité de la différenciation. Les résultats d'analyse sont souvent présentés sous forme de nuage de points en deux dimensions, sur lequel une segmentation en sous-groupes ou « clusters » est réalisée ; si les clusters se superposent partiellement, l'estimation de répartition entre les sous-groupes peut être faussée.

Conscients des limites de ces systèmes, de nombreux acteurs du domaine ne les utilisent qu'en première étape, pour « filtrer » les échantillons problématiques. Par la suite, ils procèdent souvent à une analyse visuelle complémentaire ou « revue de lame », par un opérateur, ceci sur une part non négligeable des échantillons de sang à analyser pouvant aller jusqu'à 30% des échantillons.

Cette situation est insatisfaisante en termes de coûts et de cadence, notamment si la proportion d'échantillons pathologiques est élevée, ce qui est susceptible de se produire lorsque l'analyse est réalisée à l'hôpital ou dans un laboratoire spécialisé.

Pour automatiser la « revue de lame » et limiter le besoin d'analyse supplémentaire par des opérateurs, il a été proposé de compter et de différencier les éléments d'intérêt par des mesures visuelles, à partir d'images microscopiques d'échantillons de sang sur lesquels les éléments d'intérêt sont mis en valeur.

Le document brevet WO 2010/126903 décrit une méthode comprenant l'application d'un échantillon de sang sur une lame (après coloration des leucocytes) et l'étalement de l'échantillon le long de la lame, pour créer une zone de lecture. La zone de lecture comprend, par exemple, environ 600 cellules dans le cas où les éléments d'intérêt sont des leucocytes. Des photographies de la zone de lecture sur la lame sont réalisées et font l'objet d'une analyse automatique d'images. Les photographies sont grossies au niveau des leucocytes pour détecter une taille et une forme des leucocytes, afin de différencier les leucocytes.

Les mesures visuelles permettent une caractérisation morphologique poussée des éléments d'intérêt. Il est connu de réaliser la différenciation des leucocytes (lymphocytes, monocytes, basophiles, éosinophiles et neutrophiles) de cette manière. Des mesures visuelles sont également pertinentes pour d'autres types d'éléments d'intérêt, comme les cellules érythrocytaires immatures telles que des érythroblastes ou des réticulocytes, ou encore pour détecter la présence de parasites (malaria) se caractérisant par la présence d'ADN/ARN dans les érythrocytes. La caractérisation morphologique des érythrocytes est aussi pertinente dans des cas de drépanocytose ou d'anémie.

La méthode à mesures visuelles du document WO 2010/126903 présente toutefois plusieurs inconvénients. Il est nécessaire d'obtenir et d'enregistrer de nombreuses images grossies de la lame, ce qui prend du temps pour le déplacement de la lame afin d'observer chaque cellule d'intérêt. Le temps d'analyse d'un échantillon est donc considérable, notamment s'il faut compter de nombreuses cellules d'intérêt par échantillon.

De plus, l'intérêt statistique de la répartition obtenue diminue si la concentration en leucocytes est trop basse, puisqu'on ne dispose pas de suffisamment de leucocytes sur les photographies pour déterminer une répartition fiable, ce qui réduit la précision du résultat. Si, à l'inverse, la concentration en leucocytes est trop élevée, les leucocytes peuvent se superposer sur la lame et leur taille et leur forme n'est pas correctement détectée, on perd alors en fiabilité. L'état de la technique pertinent peut également être trouvé dans le document WO 2015/001553 A1 et dans la divulgation du document de Martinazzi Massimo: "A Slide Centrifugation Technic for Concentrating Blood _eukocytes and Nucleated Cells from Bone Marrow Blood", dans American journal of clinical cathology, 1 décembre 1971.

Les moyens actuels pour automatiser la « revue de lame » ne donnent donc pas entière satisfaction.

## EXPOSE DE L'INVENTION

Les aspects de l'invention sont tels exposés dans les revendications annexées.

Il existe donc un besoin pour une analyse automatique d'échantillon de fluide corporel donnant des résultats très fiables de comptage et de différenciation des éléments d'intérêt (notamment des leucocytes), afin de réduire au maximum la nécessité d'une analyse visuelle humaine.

On cherche à obtenir des résultats fiables en évitant un fonctionnement en « boîte noire » : les résultats des mesures visuelles sur les leucocytes doivent être vérifiables par un observateur humain, pour permettre la détection d'artefacts sur la chaîne de traitement qui seraient de nature à fausser la mesure.

Il existe un besoin additionnel pour un procédé de prise de vues d'éléments d'intérêt (et notamment de leucocytes) qui s'adapte à la concentration initiale en leucocytes de l'échantillon, cette dernière étant variable et initialement inconnue. Les mesures doivent toutefois demeurer fiables.

En particulier, on souhaite obtenir des résultats précis, y compris à partir d'échantillons comportant initialement peu ou beaucoup de leucocytes en volume.

Il existe un autre besoin additionnel pour un procédé permettant de limiter le taux de revue de lame en réalisant des mesures visuelles à une cadence élevée, ne nécessitant pas d'extraire de nombreuses vues à des grossissements trop élevés.

A ce titre, l'invention concerne selon un premier aspect un procédé de prise de vue d'un échantillon de fluide corporel en vue de mesures visuelles se rapportant à des éléments d'intérêt de l'échantillon, comprenant les étapes suivantes :

une acquisition de concentration en éléments d'intérêt d'une solution issue de l'échantillon, résultant en l'obtention d'une concentration mesurée en éléments d'intérêt de l'échantillon ;

une dilution d'une solution de test issue de l'échantillon, selon un taux de dilution déterminé en fonction de la concentration mesurée en éléments d'intérêt de l'échantillon, de sorte à ramener la concentration en éléments d'intérêt de la solution de test à une concentration optimale ;

une prise de vue, par un dispositif imageur, de la solution de test transférée dans une chambre optique.

Le procédé de l'invention comprend ainsi une dilution automatique et spécifique à l'échantillon (dilution d'ajustement), basée sur la concentration en éléments d'intérêt initiale de l'échantillon, pour ajuster la concentration de la solution de test issue de l'échantillon. La prise de vue est ensuite réalisée sur la solution de test qui a fait l'objet de la dilution d'ajustement.

Cet ajustement assure que la solution de test dans la chambre optique est appropriée pour réaliser les mesures visuelles, et garantit la précision statistique et la fiabilité des mesures.

Un avantage du procédé selon l'invention est donc de limiter l'impact des variations de la concentration initiale en éléments d'intérêt de l'échantillon sur la qualité des mesures visuelles. Le procédé est particulièrement avantageux dans le cas où les éléments d'intérêt sont des leucocytes, puisque leur concentration peut varier jusqu'à un facteur 200 en fonction des pathologies. La plage des concentrations en leucocytes observables dans le sang de différents individus humains est très étendue, si bien qu'il est particulièrement pertinent de réaliser une dilution d'ajustement dans le cas où les éléments d'intérêt à observer sont des leucocytes.

Des images d'éléments d'intérêt individuels sont, par la suite, rapidement extractibles de la prise de vue de la solution de test. Le procédé de l'invention permet donc d'optimiser la cadence d'analyse en limitant le nombre de prises de vue nécessaires, la cadence étant obtenue comme un nombre d'échantillons analysés par unité de temps.

Ainsi, on peut obtenir une densité optimale d'éléments d'intérêt sur les vues tout en conservant une cadence élevée, et ce quelle que soit la concentration initiale d'éléments d'intérêt. Il est ainsi possible de réduire la proportion d'échantillons à analyser devant faire l'objet d'une « revue de lame » par un opérateur humain jusqu'à moins de 2%.

Le procédé de l'invention, associé à la dilution d'ajustement, est également avantageux pour réaliser une analyse qualitative sur l'échantillon biologique. En effet, les prises de vues effectuées permettent des mesures visuelles qualitatives sur les éléments d'intérêt ou sur d'autres éléments de l'image, par exemple une caractérisation de la qualité et/ou de l'état de santé des éléments d'intérêt et/ou des autres éléments.

Les mesures visuelles associées à la dilution d'ajustement permettent ainsi de fournir des données cliniques pertinentes, éventuellement quantitatives et qualitatives, tout en assurant une cadence optimale d'analyse et une bonne précision statistique des mesures.

Des caractéristiques additionnelles et non limitatives du procédé de prise de vue de l'invention sont les suivantes, prises seules ou en l'une des combinaisons techniquement possibles :

- le procédé comprend une étape supplémentaire, antérieure à la prise de vue, de concentration des éléments d'intérêt de la solution de test sur un même plan optique perpendiculaire à une direction d'épaisseur de la chambre optique.

- le procédé comprend une étape d'entraînement en rotation de la chambre optique par une unité de centrifugation après le transfert de la solution de test dans la chambre optique, de sorte à réaliser un alignement des éléments d'intérêt (de préférence des leucocytes) de la solution de test sur un plan optique compris dans la chambre optique, le plan optique étant perpendiculaire à une direction d'épaisseur de la chambre optique.

Une centrifugation réalisée par une unité de centrifugation permet d'aligner de manière automatique et rapide les éléments d'intérêt, de préférence les leucocytes, sur le plan optique de la chambre optique.

Grâce à la dilution d'ajustement de la solution de test (réalisée en fonction de la concentration mesurée en éléments d'intérêt dans l'échantillon initial de fluide corporel) associée à l'étape de centrifugation, il est possible de ramener la densité surfacique des éléments d'intérêt sur le plan optique à une densité surfacique cible, quelle que soit la concentration mesurée en éléments d'intérêt dans l'échantillon initial.

L'obtention d'une telle densité surfacique cible assure que le taux de recouvrement des éléments d'intérêt sur les images issues de la ou des prise(s) de vue(s) ne soit pas trop élevé, tout en assurant qu'un nombre suffisant d'éléments d'intérêt soit visible sur lesdites images. Ainsi, la pertinence des mesures visuelles ultérieures est améliorée ;

- la concentration optimale de leucocytes pour la solution de test correspond à une densité surfacique cible sur le plan optique comprise entre 30 éléments d'intérêt par millimètre carré et 1000 éléments d'intérêt par millimètre carré, à l'issue de l'alignement des éléments d'intérêt sur le plan optique.

- l'entraînement en rotation de la chambre optique est réalisé pendant une durée comprise entre 5 secondes et 5 minutes, de préférence comprise entre 10 secondes et 1 minute.

- l'acquisition comprend une mesure non visuelle sur une solution intermédiaire issue de l'échantillon, de préférence une mesure d'impédance par un micro-orifice.

- l'acquisition comprend une mesure d'absorbance à une longueur d'onde prédéterminée sur une solution intermédiaire issue de l'échantillon, ladite longueur d'onde étant de préférence de 540 nanomètres.

- le taux de dilution D est calculé à partir de la concentration mesurée WIC en éléments d'intérêt de l'échantillon à l'aide de la formule suivante :

$$D = WIC * h / WPD,$$

où h est la hauteur de la chambre optique et où WPD est la densité surfacique cible sur le plan optique PO.

- le taux de dilution D est calculé à partir de la mesure WIC de concentration en éléments d'intérêt de l'échantillon à l'aide de la formule suivante :

$$D = WIC * V / WPC,$$

où V est le volume de la chambre optique et où WPC est un nombre optimal de leucocytes sur une vue.- le procédé comprend une étape de comparaison de la concentration mesurée en éléments d'intérêt de l'échantillon par rapport à un seuil, la dilution de la solution de test issue de l'échantillon étant réalisée en fonction du résultat de ladite comparaison.

- le taux de dilution D est compris entre 10 et 1000.

- les éléments d'intérêt sont des leucocytes.

- les éléments d'intérêt sont sous-représentés dans l'échantillon (cas des leucocytes dans le sang humain), et le procédé comprend une étape supplémentaire de préparation de la solution de test comprenant une séparation cellulaire et/ou une lyse sélective chimique et/ou physique pour réaliser un tri cellulaire conservant les éléments d'intérêt.

Le procédé de l'invention, associé à une lyse des érythrocytes de l'échantillon, est particulièrement avantageux dans le contexte de l'analyse morphologique des leucocytes (les éléments d'intérêt étant alors les leucocytes), puisque les leucocytes sont 1000 fois moins nombreux que les érythrocytes ou les thrombocytes dans le sang humain.

Ainsi, grâce à la lyse des érythrocytes avant de réaliser la prise de vue de l'échantillon, les mesures visuelles subséquentes sur les leucocytes sont optimisées en termes de qualité et densité d'éléments d'intérêt. Notamment, si une densité surfacique comprise entre 20 leucocytes par millimètre carré et 1000 leucocytes par millimètre carré est obtenue sur le plan optique de la chambre optique préalablement à la prise de vue, il est avantageux de mettre en oeuvre l'étape de tri cellulaire pour éliminer les érythrocytes qui diminueraient fortement la qualité des mesures visuelles.

- l'image obtenue à l'issue de la prise de vue présente un nombre total de leucocytes compris entre 5 et 200, de préférence compris entre 140 et 160.

- l'étape de prise de vue comprend une pluralité d'acquisitions d'images à des positions distinctes du dispositif imageur par rapport à la chambre optique, le dispositif imageur se déplaçant le long d'une même direction entre deux positions consécutives.

- le procédé comprend une mesure visuelle réalisée sur l'image de la solution de test, la mesure visuelle comportant de préférence un comptage différentiel des éléments d'intérêt, un résultat dudit comptage comprenant une répartition des éléments d'intérêt de l'échantillon parmi une pluralité de sous-groupes.

- le procédé contient une étape supplémentaire de préparation de la solution de test comprenant une perméabilisation des membranes des éléments d'intérêt et/ou une coloration distinctive des éléments d'intérêt.

- les éléments d'intérêt sont des érythrocytes ou des thrombocytes.

Selon un deuxième aspect, l'invention concerne un système de prise de vues se rapportant à des éléments d'intérêt d'un échantillon de fluide corporel, comprenant :

un dispositif de mesure de concentration en éléments d'intérêt d'un échantillon de fluide corporel,

un dispositif de dilution adapté pour réaliser une dilution d'une solution issue d'un échantillon de fluide corporel selon un taux de dilution déterminé,
une chambre optique adaptée pour recevoir une solution de test issue d'un échantillon de fluide corporel,

un dispositif imageur,

un processeur configuré pour commander le dispositif imageur et pour recevoir, depuis le dispositif de mesure, une mesure de concentration en éléments d'intérêt de l'échantillon, le processeur étant en outre configuré pour calculer un taux de dilution dépendant de ladite mesure.

Des caractéristiques additionnelles et non limitatives du système de l'invention sont les suivantes, prises seules ou en combinaison :

- le système comprend en outre une unité de centrifugation configurée pour entraîner en rotation la chambre optique contenant la solution de test.

- le dispositif de mesure de concentration en éléments d'intérêt comprend une cellule à micro-orifice, adaptée pour déterminer un comptage d'éléments d'intérêt par impédance.

- le dispositif imageur comprend des moyens de déplacement adaptés pour déplacer un objectif du dispositif imageur selon un axe de lecture de la chambre optique.

- la chambre optique comprend une paroi de support de leucocytes délimitant un plan optique, la paroi de support de leucocytes présentant une épaisseur comprise entre 0,05 millimètres et 0,5 millimètres, l'unité de centrifugation étant configurée de sorte à ramener des leucocytes de la solution de test sur ledit plan optique.

Selon un troisième aspect, l'invention concerne un produit programme d'ordinateur comprenant des instructions de code qui, lorsque le programme est exécuté par un processeur, conduisent le processeur à mettre en oeuvre une commande de prise de vue par un dispositif imageur, une réception, depuis un dispositif de mesure, d'une mesure de concentration en éléments d'intérêt d'un échantillon et un calcul d'un taux de dilution en fonction de ladite mesure.

## DESCRIPTION GENERALE DES FIGURES

D'autres caractéristiques, buts et avantages de l'invention ressortiront de la description qui suit, qui est purement illustrative et non limitative, qui doit être lue en regard des dessins annexés parmi lesquels :

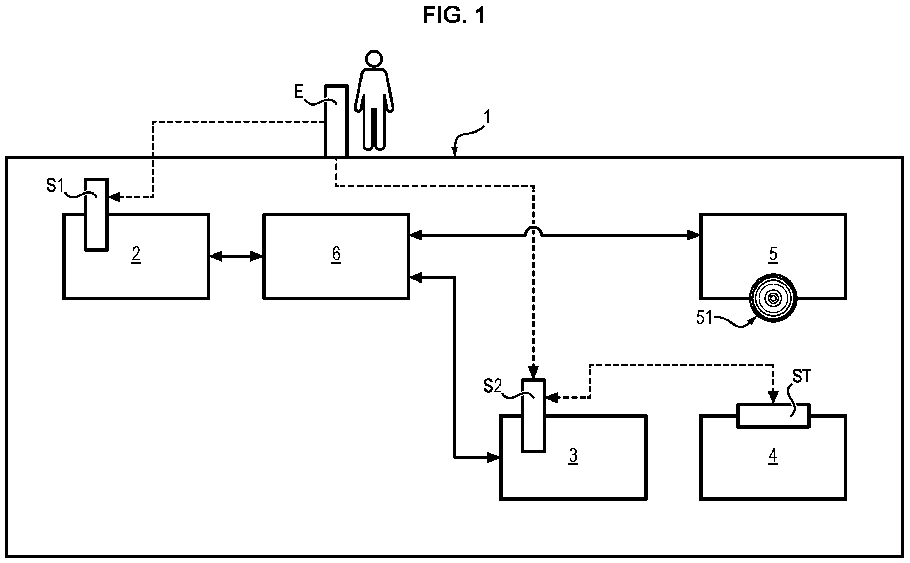

La **figure 1** représente schématiquement un système de prise de vues sur des échantillons de fluides corporels tels que des échantillons de sang, selon un mode de réalisation de l'invention ;

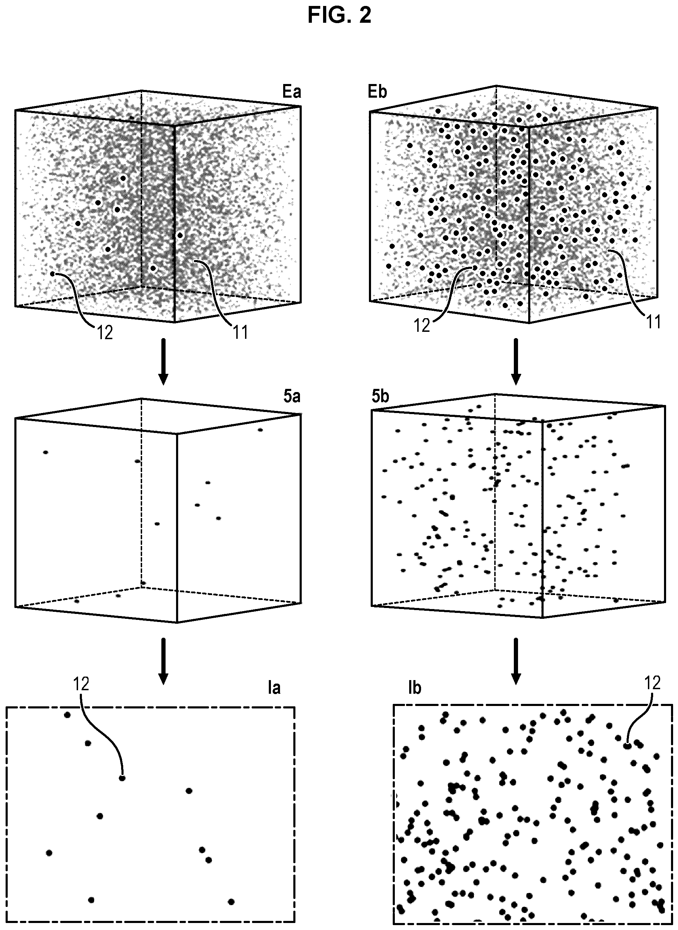

La **figure 2** illustre des étapes de lyse et de centrifugation d'un échantillon de sang. La colonne de gauche se rapporte à un échantillon de faible concentration en leucocytes. La colonne de droite se rapporte à un échantillon de concentration élevée en leucocytes ;

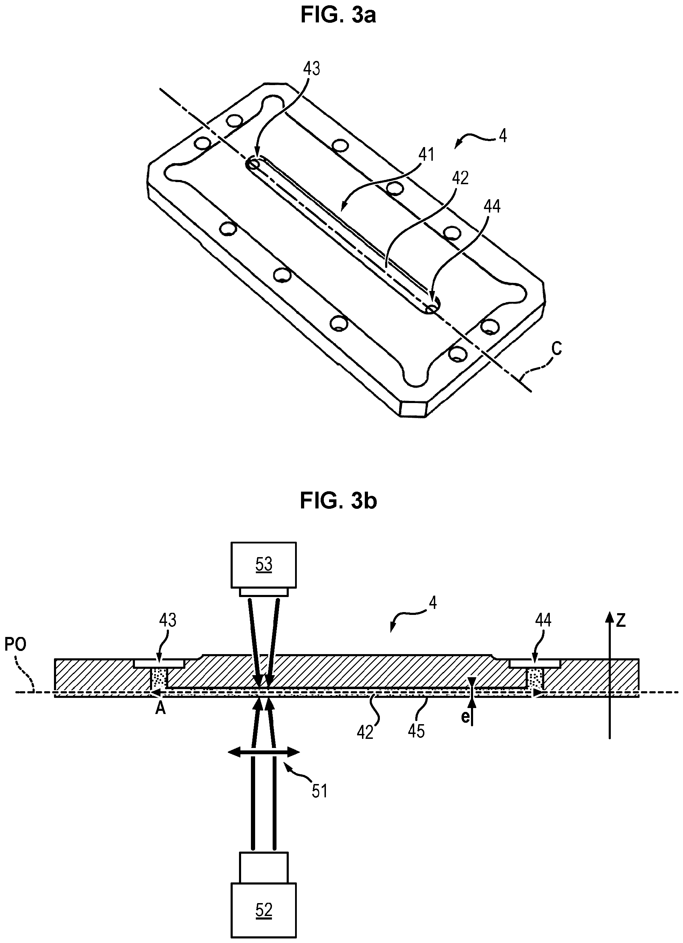

La **figure 3a** est une vue de dessus en perspective d'une chambre optique du système de la Figure 1 ;

La **figure 3b** est une vue schématique de côté de la même chambre optique associée à un dispositif de prise de vues ;

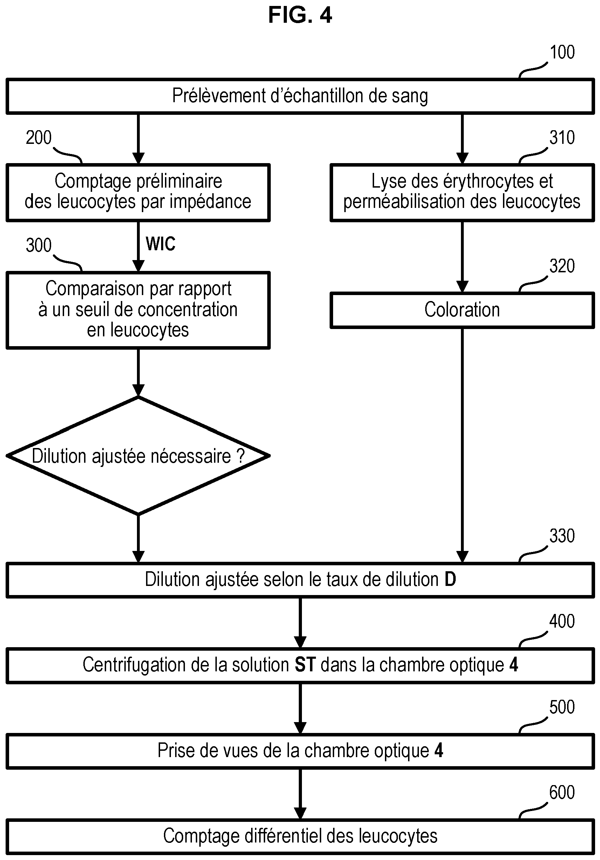

La **figure 4** illustre des étapes d'un procédé de prise de vues et de mesures visuelles sur un échantillon de sang, selon un mode de réalisation de l'invention ;

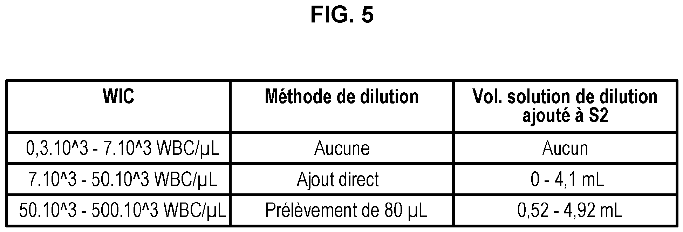

La **figure 5** est un tableau comparatif des protocoles de dilution adaptés pour diverses concentrations en cellules d'intérêt de l'échantillon de sang ;

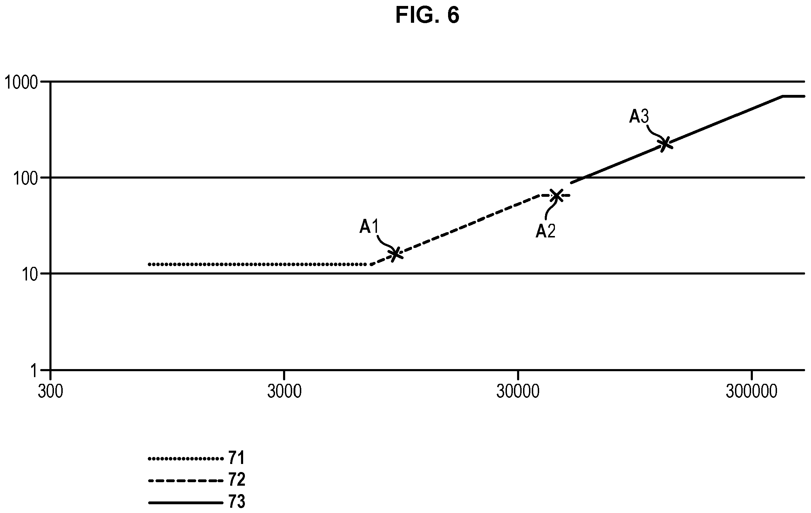

La **figure 6** est un graphique qui représente en ordonnée le taux de dilution appliqué pour réaliser l'ajustement de concentration souhaité et obtenir la solution de test, et en abscisse la concentration mesurée en leucocytes dans un échantillon initial de sang, selon un exemple de réalisation ;

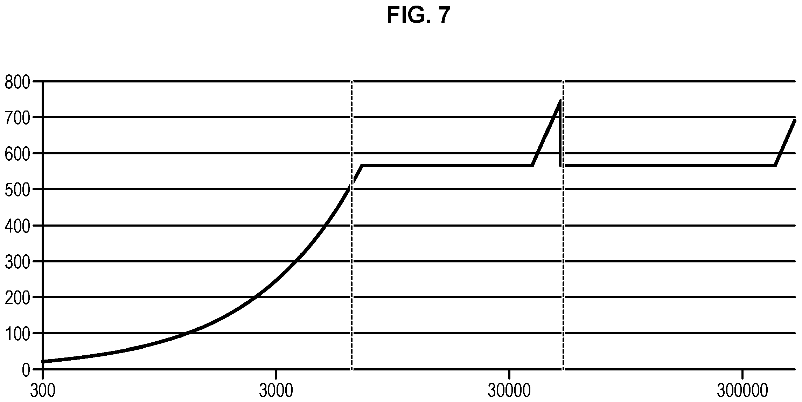

La **figure 7** est un graphique qui représente en ordonnée la densité surfacique en leucocytes par millimètre carré obtenue sur le plan optique de la chambre optique à l'issue de la centrifugation de la solution de test, et en abscisse la concentration mesurée en leucocytes dans l'échantillon initial, selon le même exemple de réalisation ;

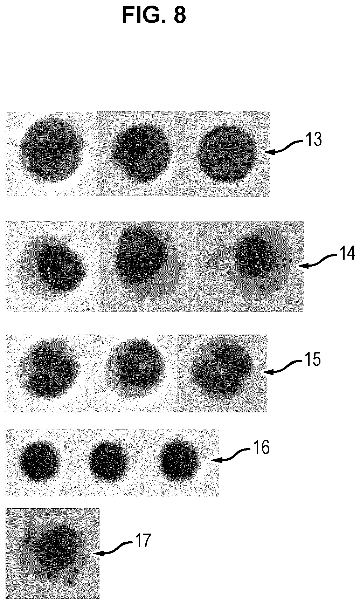

La **figure 8** est une image de divers sous-groupes de leucocytes ayant fait l'objet d'une coloration dans un échantillon de sang ;

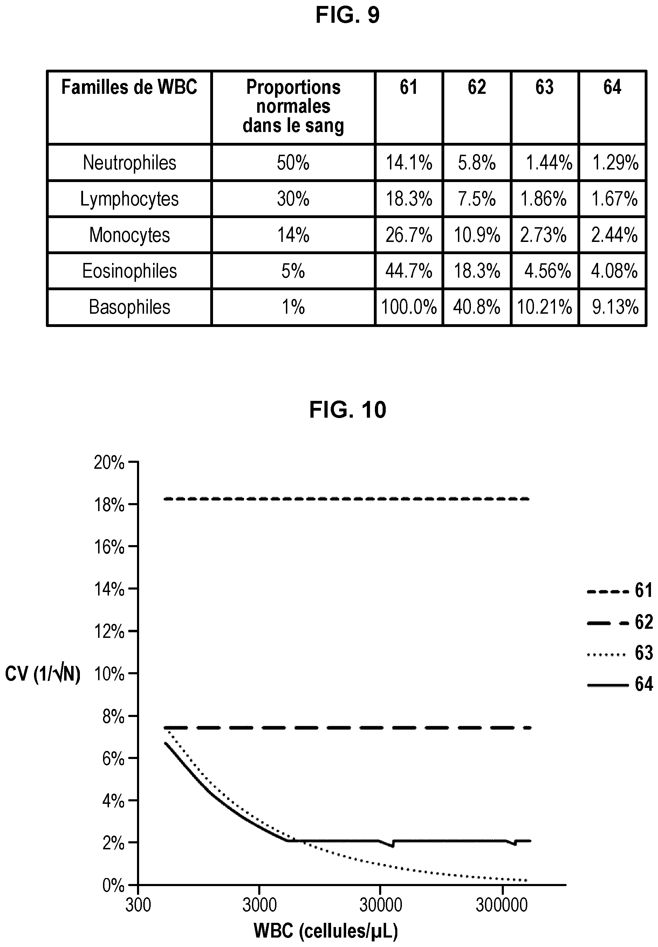

La **figure 9** est un tableau comprenant des données de performance de mesure de différenciation de leucocytes, en fonction des sous-groupes de leucocytes et en fonction du type de mesure utilisé pour la différenciation ;

La **figure 10** est un graphique représentatif de la performance statistique de différenciation leucocytaire (en ordonnée) uniquement pour le sous-groupe des lymphocytes, en fonction de la concentration en lymphocytes de l'échantillon initial (en abscisse) et en fonction du type de mesure utilisé pour la différenciation ;

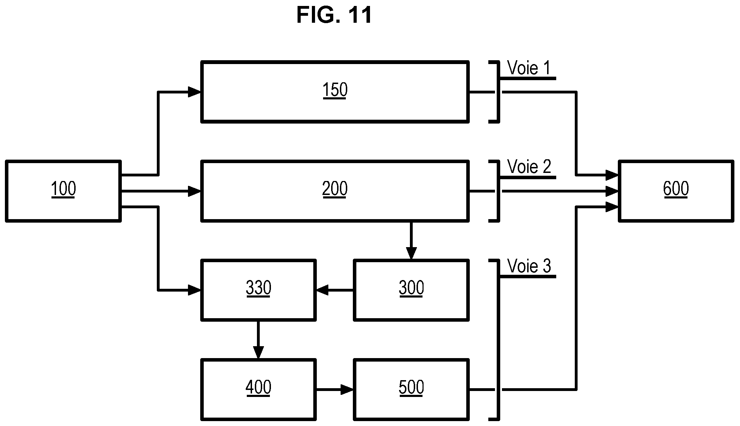

La **figure 11** représente les étapes correspondant à trois voies de mesure pour l'analyse d'un échantillon de sang.

## DESCRIPTION DETAILLEE DE L'INVENTION

La description ci-après concerne principalement la préparation d'une solution d'hématologie à partir d'un échantillon de sang, pour réaliser des mesures de différenciation et de morphologie d'éléments d'intérêt, ici des leucocytes. Toutefois, l'invention s'applique également pour tout échantillon de fluide, notamment de fluide corporel, comprenant des éléments d'intérêt sur lesquels on souhaite réaliser des mesures visuelles comme des cellules, cristaux, parasites etc... Ces éléments d'intérêt sont typiquement en suspension dans les fluides corporels comme le sang, l'urine, le liquide lymphatique, le liquide amniotique, etc... Les éléments d'intérêt sont pathologiques (blastes, lymphomes...) ou non pathologiques.

Dans toute la suite, des « mesures visuelles » sont des mesures réalisées à partir d'images acquises sur une solution issue de l'échantillon, sur lesquelles des éléments d'intérêt (éventuellement marquées) sont visibles. Les mesures visuelles sont réalisées à partir d'une ou plusieurs images de la solution, de préférence de manière automatique, par analyse d'images.

Dans la description ci-après et sur les figures annexées, des éléments similaires sont désignés par les mêmes références alphanumériques.

### Système de préparation de solution hématologique et de prise de vues

On a représenté en **Figure 1** un système 1 permettant de préparer une solution de test ST et d'acquérir des vues de la solution de test ST, à partir d'un échantillon E de sang prélevé sur un individu, par exemple en hématologie. Le système 1 de la Figure 1 peut être utilisé dans un laboratoire d'analyse médicale, pour obtenir des résultats aidant au diagnostic d'une éventuelle pathologie de l'individu.

Le système 1 comprend un dispositif de mesure 2 réalisant des mesures d'hématologie. Le dispositif de mesure 2 est configuré pour acquérir des mesures hématologiques préliminaires sur une solution intermédiaire S1 issue de l'échantillon E. Les mesures préliminaires comprennent une concentration en leucocytes. La solution S1 est par exemple issue d'une dilution de l'échantillon E.

De préférence, le dispositif de mesure 2 comprend une cellule comportant un orifice de $100\mu m$ de diamètre, dit « micro-orifice », la cellule à micro-orifice étant configurée pour réaliser un comptage par impédance des leucocytes. Le comptage par impédance constitue une voie de mesure non visuelle de la concentration en leucocytes de la solution intermédiaire S1. Un avantage de la mesure par impédance est sa cadence élevée due au traitement de signal analogique réalisé par un microprocesseur dédié.

A partir de la concentration en leucocytes de la solution S1, et en connaissant le taux de dilution entre l'échantillon E et la solution S1, on peut obtenir, à l'aide du dispositif de mesure 2, une mesure WIC (pour « White blood cells Impedance Count ») de la concentration en leucocytes dans l'échantillon E.

La mesure WIC donne un résultat considéré fiable pour le comptage d'éléments d'intérêt (ici les leucocytes). Toutefois, les résultats de différenciation fournis par un comptage par impédance sont considérés comme non satisfaisants pour les raisons exposées ci-avant en introduction.

Le système de la Figure 1 comprend en outre un dispositif de dilution 3, une chambre optique 4 et un dispositif photographique 5.

Le dispositif de dilution 3 est adapté pour réaliser une dilution d'un échantillon de fluide, par addition de solution de dilution à un volume déterminé de solution intermédiaire S2 issue de l'échantillon E. Ladite solution intermédiaire S2 est issue de la préparation chimique et/ou physique de l'échantillon E - la préparation chimique et/ou physique étant réalisée, selon une variante possible, en parallèle de la dilution permettant d'obtenir la solution S1. Le dispositif de dilution 3 permet ici de réaliser la dilution de l'échantillon E pour obtenir une solution intermédiaire S2, puis une dilution d'ajustement de la solution S2 pour obtenir une solution de test ST.

En alternative, la première dilution pour obtenir la solution intermédiaire S2 à partir de l'échantillon E est réalisée par un dispositif différent de celui dédié à la dilution d'ajustement pour obtenir la solution de test ST à partir de la solution intermédiaire S2. De façon avantageuse, le taux de dilution appliqué à la solution S2 est choisi en fonction d'une concentration optimale en leucocytes souhaitée pour la solution de test ST.

Le système 1 comprend en outre une unité de traitement 6. On notera que dans l'exemple de la Figure 1, les dispositifs 2, 3, 4 et 5 et l'unité de traitement 6 sont compris dans une même enceinte du système 1. Le système 1 est par exemple un automate de diagnostic.

On compare en **Figure 2** des résultats de prise de vues obtenus pour un échantillon de sang de faible concentration en leucocytes 12 (à gauche) et pour un échantillon de concentration élevée en leucocytes 12 (à droite). Aucune dilution d'ajustement n'a été réalisée. Autrement dit, la Figure 2 concerne des échantillons pour lesquels le taux de dilution n'a pas été ajusté en fonction de la concentration initiale en leucocytes. Les échantillons Ea et Eb comprennent en outre des érythrocytes 11. En moyenne, la concentration en érythrocytes est 1000 fois supérieure à la concentration en leucocytes.

On a représenté les solutions Sa et Sb obtenues à l'issue d'une lyse des érythrocytes, respectivement à partir des échantillons Ea et Eb, ainsi que les images Ia et Ib acquises respectivement à partir des solutions Sa et Sb. Sur l'image Ia, on ne dispose pas de suffisamment de leucocytes pour réaliser un comptage différentiel fiable des leucocytes. A l'inverse, la concentration en leucocytes est trop élevée sur l'image Ib et les leucocytes se superposent par endroits, faussant le comptage et les mesures morphologiques. En ajustant la concentration en leucocytes (de préférence conjointement à une lyse des érythrocytes), on peut donc améliorer et faciliter les mesures visuelles réalisées ultérieurement sur les prises de vues.

Le dispositif de dilution 3 permet d'ajouter de la solution de dilution (dans l'exemple ci-après, du diluent comprenant par exemple un agent de lyse et/ou un agent colorant) à un échantillon de liquide.

Il est pertinent d'ajouter de l'agent de lyse au moment d'une dilution d'un échantillon biologique, au cas où toutes les cellules qu'on a souhaité éliminer par une précédente lyse n'auraient pas été correctement éliminées. En effet, l'ajout de diluent peut faire réapparaître des résidus de cellules non correctement lysées.

Ici, le dispositif de dilution 3 comprend un récipient présentant une ouverture à une première extrémité pour recevoir un volume de sang et un volume de diluent, et présentant un tube à une deuxième extrémité pour injecter des bulles dans le récipient et réaliser une agitation du mélange sang/diluent. Le dispositif de dilution 3 comprend ici un tube supplémentaire pour extraire le mélange sang/diluent après dilution. Dans le présent exemple, un volume maximal de fluide contenu dans le récipient est de 5 millilitres.

Alternativement, les dispositifs 3 et 4 peuvent être utilisés pour l'observation d'érythrocytes (et non de leucocytes uniquement), auquel cas la solution intermédiaire S2 serait préparée différemment à partir de l'échantillon E.

La chambre optique 4 est représentée sur la **Figure 3a** (sans son couvercle) en vue en perspective de dessus. La chambre optique 4 est représentée sur la **Figure 3b** en coupe longitudinale selon le plan C représenté sur la Figure 3a.

La chambre optique 4 comprend deux ouvertures circulaires 43 et 44 entre lesquelles s'étend un canal de lecture 42. Les ouvertures 43 et 44 permettent le transfert de fluide depuis l'extérieur. Le canal de lecture 42 comprend deux portions verticales à l'aplomb des ouvertures 43 et 44, ces deux portions étant jointes par une portion horizontale du canal 42 qui s'étend le long d'un axe de lecture A de la chambre optique. La portion horizontale présente de préférence une épaisseur e comprise entre 0,05 millimètres et 10 millimètres, par exemple entre 0,1 millimètres et 10 millimètres, par exemple de 1 millimètre. L'ouverture 43 constitue ainsi un port d'entrée et l'ouverture 44 constitue un port de sortie pour un volume de solution contenu dans le canal de lecture 42.

La chambre optique 4 est fermée sur le dessus par une face haute 41 et sur le dessous par une paroi optique fine. Les faces haute et basse sont transparentes à la lumière visible le long du canal de lecture 42. Pour fermer la chambre optique, un couvercle (non représenté) est optionnellement positionné contre la paroi optique.

Une hauteur totale de la chambre optique 4 selon la direction Z visible sur la Figure 3b est de préférence comprise entre 500 et 1500 micromètres, ladite direction Z étant perpendiculaire au plan optique PO. Dans le présent exemple, la hauteur totale de la chambre optique 4 selon la direction Z est égale à 1000 micromètres.

Le dispositif photographique 5 comprend une chambre 52 équipée d'un capteur photographique et comprend un objectif 51. Ce dispositif est utilisé avec une source de lumière 53. Le dispositif photographique 5 comprend une mémoire pour enregistrer des images $I_n$ acquises, les images étant transmises à l'unité de traitement 6.

Dans le présent exemple, l'objectif 51 est un objectif à forte correction achromatique et de grossissement 20x. De manière générale, un objectif 51 présentant un grossissement compris entre 10x et 50x est considéré comme adapté pour obtenir un nombre satisfaisant d'éléments d'intérêt par image, comme il sera vu ci-après.

Par ailleurs, l'objectif 51 présente de préférence une ouverture numérique comprise entre 0,4 et 0,6. Dans le présent exemple, ladite ouverture numérique est de 0,5.

Une fois que la solution de test ST est transférée dans le canal de lecture et prête à être photographiée, le dispositif photographique 5 est disposé à proximité de la chambre optique 4 de sorte à réaliser des prises de vues du volume interne du canal de lecture 42.

Le dispositif photographique 5 est ici positionné sous le canal de lecture 42, l'objectif 51 étant dirigé vers le canal de lecture. La source de lumière 53 est placée derrière le canal de lecture, de l'autre côté de la chambre optique. De préférence, la source de lumière 53 émet une combinaison de lumières monochromatiques ou bien une lumière blanche.

On a représenté sur la Figure 3b un plan optique PO passant par la paroi 45, la paroi 45 délimitant le bas du canal de lecture 42.

De façon très avantageuse, pour améliorer la visibilité des éléments d'intérêt sur les images acquises par le dispositif photographique 5, on prépare la solution de test ST avant la prise de vues, de sorte à aligner les éléments d'intérêt sur le plan optique PO perpendiculaire à la direction Z d'épaisseur du canal 42.

La paroi 45 délimitant le bas du canal 42 sert donc de paroi de support des cellules d'intérêt (c'est-à-dire ici des leucocytes) après préparation de la solution de test ST.

Le système de préparation et d'imagerie de la solution de test comprend ainsi un dispositif de déplacement des éléments d'intérêt de la solution de test au niveau du plan optique PO. Ici, la chambre optique 4 est propre à être entraînée par une unité de centrifugation (non représentée) permettant l'accélération de la sédimentation naturelle suivant l'axe de la mesure optique, pour ramener des cellules d'intérêt (telles que des leucocytes) de la solution de test ST contre la paroi 45.

L'unité de centrifugation est de préférence configurée pour entraîner en rotation la chambre optique 4 autour d'un axe de rotation parallèle au plan optique PO. On peut utiliser, de manière alternative ou en combinaison, comme dispositif destiné à aligner les éléments d'intérêt sur le plan optique PO, un dispositif de filtration ou un dispositif microfluidique.

De préférence, la paroi 45 présente une épaisseur selon la direction Z comprise entre 0,05 millimètres et 0,5 millimètres. De manière encore préférentielle, ladite épaisseur est comprise entre 0,1 millimètres et 0,3 millimètres, et elle est ici égale à 0,2 millimètres.

Avec une valeur d'épaisseur voisine de 0,2 millimètres, la profondeur de champ (dite DOF pour « depth of field ») de l'objectif 51 du dispositif photographique 5 peut être choisie entre 2,7 et 3,0 micromètres.

De préférence, le dispositif photographique 5 comprend en outre des moyens de déplacement (non représentés) adaptés pour déplacer l'objectif 51 et la chambre 52 selon une direction située à la verticale de l'axe de lecture A. Il peut s'agir d'un rail. Le champ visuel de l'objectif 51 est déplacé le long de l'axe A. Un avantage de cette configuration est de permettre l'acquisition d'une pluralité de vues qui couvrent tout le canal de lecture 42.

Le dispositif photographique 5 comprend aussi de préférence des moyens de déplacement de l'objectif 51 selon la direction Z d'épaisseur du canal 42, pour améliorer la mise au point.

De retour à la Figure 1, l'unité de traitement 6 est configurée pour commander le dispositif photographique 5, par exemple un serveur. L'unité de traitement 6 envoie des instructions de prise de vue, et éventuellement de déplacement, au dispositif photographique 5. L'unité de traitement comprend en outre une mémoire pour stocker les images $I_n$ fournies par le dispositif 5.

L'unité de traitement 6 est en outre configurée pour recevoir une mesure de concentration en leucocytes de l'échantillon E depuis le dispositif de mesure 2, ou en alternative, pour déterminer cette mesure à partir d'une mesure préliminaire fournie par le dispositif de mesure 2. L'unité de traitement 6 est également configurée pour calculer un taux de dilution optimal D à partir de la mesure de concentration en leucocytes de l'échantillon, selon les modalités décrites ci-après.

L'unité de traitement 6 est configurée avec des instructions de code de programme informatique pour réaliser les fonctions relevées ci-avant.

De préférence, l'unité de traitement 6 comprend également des moyens de calcul configurés pour réaliser des mesures visuelles à partir des vues I acquises sur la solution de test ST. Dans le présent exemple, les moyens de calcul sont configurés pour déterminer, à partir des images I, plusieurs paramètres d'intérêt :

- un comptage des leucocytes, c'est-à-dire une concentration totale en leucocytes ;

- un comptage différentiel (ou différenciation) des leucocytes, c'est-à-dire une répartition des leucocytes dans les cinq sous-groupes : lymphocytes, monocytes, basophiles, éosinophiles et neutrophiles ;

- des caractéristiques morphologiques des leucocytes, par exemple des informations sur la structure membranaire des leucocytes appartenant aux divers sous-groupes ou sur la structure du ou des noyaux ;

- une détection d'interférences liés à la méthode, comme une détection d'érythrocytes qui indiquerait par exemple une résistance potentielle à la lyse utilisée lors de la méthode.

En alternative, l'unité de traitement 6 est reliée, par liaison avec ou sans fil, à une unité de calcul configurée pour réaliser les mesures visuelles sur les leucocytes.

La mesure hématologique fournie par le dispositif 2 et la mesure visuelle obtenue à partir des vues $I_n$ correspondent respectivement à deux voies de mesure séparées.

Le système de la Figure 1 peut, de façon optionnelle, être complété par des d'autres voies de mesure. Les voies de mesure supplémentaires peuvent correspondre à d'autres types de éléments d'intérêt, notamment à des érythrocytes ; par exemple, le dispositif 2 peut être une cellule comportant un orifice de 78 $\mu$m de diamètre, configurée pour réaliser un comptage par impédance des érythrocytes et thrombocytes.

*Procédé de préparation de solution de test et de prise de vues*

La **Figure 4** représente les étapes d'un procédé de prise de vue à partir d'un échantillon de sang, en vue de réaliser des mesures visuelles sur les leucocytes, selon un mode de réalisation de l'invention.

Le système de préparation d'échantillons et de prise de vues de la Figure 1 est adapté pour mettre en oeuvre le procédé de la Figure 4.

De manière préférée, toutes les étapes de ce procédé - sauf éventuellement l'étape préliminaire 100 de prélèvement d'échantillon de sang - sont réalisées de manière automatique par le système 1. Comme indiqué ci-avant, le système 1 est de préférence un automate de diagnostic.

Ainsi, l'échantillon E de sang fourni au système 1 peut être un échantillon de sang pur. Une fois l'échantillon E fourni au système 1, une intervention humaine n'est de préférence pas nécessaire jusqu'à l'obtention des prises de vues. Le dispositif de mesure 2, le dispositif de dilution 3, la chambre optique 4 et le dispositif imageur 5 sont de préférence contrôlés pour mettre en oeuvre de manière coordonnée le procédé de la Figure 4.

La préparation de la solution de test ST décrite ci-après, comprenant notamment une dilution d'ajustement à l'étape 330, ne nécessite pas d'intervention humaine. Le dispositif de dilution 3 est configuré pour optimiser automatiquement la dilution réalisée, en fonction d'une concentration en leucocytes mesurée dans l'échantillon E, comme il sera vu ci-après.

A une étape préliminaire 100, un échantillon de sang E est prélevé sur un individu. On comprendra que les étapes suivantes peuvent être réalisées à un moment et en un lieu distinct de celui du prélèvement.

Conformément à l'invention, une acquisition de concentration en éléments d'intérêt de l'échantillon E est réalisée à une étape 200.

On parle ici d'acquisition donnant une mesure préliminaire de concentration en éléments d'intérêt (ici les leucocytes), car cette mesure est faite préalablement aux mesures visuelles pour la différenciation des leucocytes.

A titre d'exemple, l'étape 200 est ici une mesure d'impédance, pour obtenir un comptage des leucocytes. La mesure d'impédance est réalisée ici sur une solution intermédiaire S1, obtenue par dilution de l'échantillon E (pour obtenir environ 1600 microlitres de solution diluée) puis par ajout d'un volume de solution de lyse des érythrocytes (par exemple 200 microlitres de la solution de lyse).

La solution de lyse contient avantageusement un composé additionnel sans cyanure pour la quantification de l'hémoglobine. Ce composé forme des complexes avec l'hémoglobine de la solution. Une mesure d'absorbance, par exemple à 540 nm, permet d'évaluer la quantité de complexes ainsi formés.

L'étape 200 fournit une mesure préliminaire WIC (pour « White blood cells Impedance Count ») de la concentration en leucocytes dans l'échantillon E, en nombre de cellules par microlitre.

Comme indiqué ci-avant en relation à la Figure 2, la performance des mesures visuelles varie en fonction de la concentration en leucocytes de l'échantillon E.

Pour un échantillon non pathologique, dont l'activité des leucocytes peut être considérée comme normale, il est estimé que la concentration mesurée WIC est comprise entre 3500 et 11000 leucocytes par microlitre, pour un être humain.

Il a été observé que, dans le cas général, cette mesure préliminaire WIC peut varier entre 300 et 500000 leucocytes par microlitre, lorsque les échantillons pathologiques sont également inclus.

De ce fait, il convient de déterminer s'il est nécessaire de réaliser une dilution d'ajustement de la solution de test, afin d'optimiser la concentration en leucocytes de la solution de test.

De manière préférentielle, on souhaite obtenir une certaine densité surfacique cible en éléments d'intérêt (ici en leucocytes) sur un plan optique photographié lors des prises de vues, quelle que soit la concentration initiale en éléments d'intérêt dans l'échantillon E. Comme il sera vu ci-après, une densité surfacique cible en leucocytes dans le plan optique PO, dite WPD, est de préférence comprise entre 20 et 1000 leucocytes par millimètre carré, et est ici égale à 570 leucocytes par millimètre carré.

Ici, à une étape 300, la mesure WIC est comparée à un seuil de concentration en leucocytes de l'échantillon E. Ce seuil correspond avantageusement à une quantité optimale d'éléments d'intérêt sur une image acquise par le dispositif 5 ; ici, la quantité voulue sur une image est déterminée par la taille du capteur choisi.

Dans le système de la Figure 1, le champ visuel de l'objectif 51 est de largeur 0,5 millimètre et de longueur 0,7 millimètre.

L'épaisseur du canal de lecture 42 de la chambre optique 4 est choisie en fonction du nombre de leucocytes souhaité par image (à l'issue de l'étape de prise de vue 500 qui sera décrite ci-après). La concentration en leucocytes de la solution de test ST sur laquelle les prises de vues sont réalisées est exprimée en nombre de leucocytes par microlitre.

A raison d'une dilution par défaut à 1/12 entre l'échantillon E et la solution de test ST, on obtient un seuil maximum à 5000 cellules par microlitre pour l'échantillon E.

Dans le présent exemple, si la concentration en leucocytes de l'échantillon E est supérieure au seuil maximum, il est déterminé qu'une dilution d'ajustement est préférable. Par « dilution d'ajustement » on entend que le taux de dilution D, et donc le volume de solution de dilution (ici le diluent) à ajouter, est déterminé en fonction de la concentration mesurée WIC en leucocytes dans l'échantillon E pour ajuster la concentration de la solution de test (ST).

On peut aussi comparer la mesure WIC à un seuil maximal au-delà duquel on considère que le risque d'obtenir des vues saturées en leucocytes est élevé. Dans ce dernier cas, les zones comportant des recouvrements d'éléments d'intérêt sur les images sont exclues de la prise de vues.

La préparation de la solution de test ST destinée à la prise de vues dépend donc du résultat de la comparaison réalisée à l'étape 300.

Si la mesure préliminaire WIC est au-dessus du seuil maximum de cellules par microlitre, la solution de test ST est obtenue en réalisant une dilution d'ajustement, à partir d'une solution intermédiaire S2 issue de l'échantillon E.

On exploite ainsi, de manière habile, la mesure d'impédance fournie par le dispositif 2 - qui est une mesure non visuelle - pour préparer la solution de test ST avant de réaliser les mesures visuelles. Pour préparer la solution de test ST à partir de l'échantillon E, on prépare de préférence, au cours d'une étape 310, une solution intermédiaire S2. Pour obtenir la solution intermédiaire S2, on ajoute dans un premier temps à un volume de sang extrait de l'échantillon E un volume de solution de lyse.

Par exemple, la solution de lyse comprend une solution aqueuse de tampon neutre, à une concentration hypotonique. Une lyse des érythrocytes est ainsi réalisée.

On peut ajouter un agent de solubilisation des membranes des érythrocytes, et/ou un agent de perméabilisation des membranes cellulaires des leucocytes.

Un avantage de la lyse sélective des érythrocytes est de permettre que la majorité des cellules sanguines visibles sur les prises de vues soient des leucocytes.

De préférence, le taux d'érythrocytes dans les images issues des prises de vues (parmi le nombre total de cellules visibles) est inférieur à 10%. De manière plus préférentielle, le taux d'érythrocytes est approximativement nul, et seuls quelques érythrocytes très isolés demeurent visibles sur les images issues des prises de vues.

Dans le cas où l'on souhaite visualiser les érythrocytes en tant que cellules d'intérêt, à la place des leucocytes, l'étape 310 de lyse est omise.

Pour mettre en valeur les éléments d'intérêt, on réalise de préférence, à une étape 320, une coloration de la solution obtenue après lyse des érythrocytes. Dans le présent exemple, on combine un ou plusieurs colorants anioniques et un ou plusieurs colorants cationiques. On peut également utiliser isolément l'un ou l'autre de ces colorants. Ces colorants sont utilisés de manière séquentielle pour se fixer sur les éléments d'intérêt, dans l'ordre de préférence anionique puis cationique.

De manière préférentielle, les étapes 310 et 320 de préparation de la solution de test ST à partir de l'échantillon E initial ne comprennent pas l'utilisation de marqueurs de fluorescence. En effet, grâce à la dilution d'ajustement de la solution de test ST décrite ci-après, la visibilité des leucocytes sur les images issues de la prise de vues est satisfaisante, sans qu'il ne soit indispensable d'utiliser de tels marqueurs.

A l'issue des étapes 310 et 320, on obtient la solution intermédiaire S2. S'il a été déterminé à l'étape 300 qu'il est nécessaire d'ajuster la dilution pour obtenir la solution de test ST, on réalise à l'étape 330 une dilution d'ajustement de la solution intermédiaire S2, à l'aide du dispositif de dilution 3.

De façon avantageuse, le taux de dilution D utilisé pour passer de la solution intermédiaire S2 à la solution de test ST est calculé à partir de la mesure WIC de concentration en leucocytes obtenue à l'issue de l'étape 200, à l'aide de la formule :

$$D = WIC * h/ WPD,$$

où h est la hauteur de la chambre optique 4, et où WPD (pour « White blood cells Picture Density ») est une densité cible de leucocytes par unité de surface sur le plan optique PO où sont acquises les images $I_n$.

La hauteur h, correspondant à l'épaisseur de la chambre optique 4, est par exemple de 1 millimètre.

A titre d'exemple, une équation du taux de dilution D en fonction de la mesure WIC de concentration en leucocytes (en leucocytes par microlitre) est comme suit :

$$D = 0,0018 * WIC.$$

Dans l'exemple de la Figure 6 décrite ci-après, le taux de dilution est déterminé selon l'équation D = 0,0018 * WIC lorsque la concentration mesurée WIC en leucocytes de l'échantillon E initial est supérieure ou égale à 7000 leucocytes par microlitre.

La dilution d'ajustement 330 est réalisée par ajout d'un volume de diluent déterminé en fonction du taux de dilution D.

Le tableau de la **Figure 5** donne, pour un exemple, en fonction de la mesure préliminaire WIC de concentration en leucocytes de l'échantillon E (colonne de gauche) en nombre de leucocytes par microlitre :

- la méthode préférentielle de dilution utilisée à l'étape 330 : ajout direct de solution de dilution, ou prélèvement préalable d'un volume réduit de la solution intermédiaire S2 et ajout de solution de dilution dans ce volume réduit ;

- la plage de volume de solution de dilution à ajouter. Ce volume dépend directement du taux de dilution D déterminé conformément à la formule ci-avant.

Par exemple, pour une mesure WIC de concentration en leucocytes de l'échantillon E égale à 8000 cellules par microlitre, on ajoute un volume de diluent égal à 180 microlitres pour passer de la solution intermédiaire S2 à la solution ST.

De préférence, si la mesure WIC de concentration en leucocytes de l'échantillon E est supérieure à 38 000 cellules par microlitre, on réalise une dilution selon un taux de dilution variable à partir d'un volume extrait de la solution S2. En effet, pour une telle concentration, un ajout de diluent directement dans la solution S2 ne suffira pas à obtenir des prises de vues optimales, et un échantillonnage de S2 doit être fait. Comme décrit sur la Figure 5, pour une concentration en leucocytes inférieure au seuil maximal de cellules par microlitre (7000 leucocytes par microlitre dans l'échantillon E), de préférence, la solution intermédiaire S2 lysée et colorée est directement utilisée comme solution de test ST sans dilution additionnelle.

La **Figure 6** illustre un exemple dans lequel le taux de dilution D est calculé à l'étape 330 en fonction de la concentration mesurée WIC en leucocytes de l'échantillon E initial (en leucocytes par microlitre), selon l'équation D = 0,0018 * WIC.

Un premier tronçon 71 de la courbe représentative du taux de dilution D correspond à un premier mode opératoire, dans lequel la solution de test ST est obtenue sans dilution supplémentaire de la solution intermédiaire S2, hormis celles faisant suite aux étapes de préparation de l'échantillon E, par exemple une lyse sélective des érythrocytes et/ou une coloration des leucocytes. De préférence, aucune dilution supplémentaire n'est réalisée pour une concentration mesurée WIC en leucocytes de l'échantillon E comprise entre 300 leucocytes par microlitre et 7000 leucocytes par microlitre.

Un deuxième tronçon 72 de la courbe correspond à un deuxième mode opératoire, dans lequel la solution de test ST est obtenue par une seule dilution directe, à partir de l'échantillon E ou à partir d'une solution intermédiaire. De préférence, une seule dilution directe est réalisée pour une concentration mesurée WIC en leucocytes de l'échantillon E comprise entre 7000 leucocytes par microlitre et 50000 leucocytes par microlitre.

Un troisième tronçon 73 de la courbe correspond à un troisième mode opératoire, dans lequel la solution de test ST est obtenue par un échantillonnage de la solution intermédiaire S2 préparée à partir de l'échantillon E. De préférence, ce troisième mode opératoire est réalisé pour une concentration mesurée WIC en leucocytes de l'échantillon E dépassant 50000 leucocytes par microlitre.

Ainsi, la dilution à l'étape 330 réalise un ajustement automatique de la concentration en leucocytes, au cours de la préparation de la solution de test ST destinée à la prise de vues. En appliquant un taux de dilution D calculé en fonction de la mesure WIC, par exemple selon le calcul présenté ci-avant, il est possible d'obtenir une concentration en leucocytes de la solution de test ST optimale pour des mesures visuelles.

Une concentration en leucocytes de la solution de test ST optimale correspond à une densité surfacique d'éléments d'intérêt sur le plan optique PO de la chambre optique 4 adaptée pour bien distinguer les leucocytes et réaliser des mesures visuelles pertinentes, à l'issue de l'étape de centrifugation 400 (décrite ci-après).

De préférence, la dilution à l'étape 330 selon le taux de dilution D (comprenant éventuellement une dilution d'ajustement) permet ultérieurement d'obtenir une densité surfacique cible en leucocytes sur le plan optique PO comprise entre 20 et 1000 leucocytes par millimètre carré, une fois que les leucocytes ont été déplacés sur le plan optique PO.

De manière encore préférentielle, la densité surfacique cible en leucocytes obtenue sur le plan optique PO est comprise entre 24 et 800 leucocytes par millimètre carré.

La **Figure 7** comprend une courbe 81 représentative de la densité surfacique en leucocytes obtenue sur le plan optique PO de la chambre optique 4 (par exemple à l'issue de la centrifugation de l'étape 400 du procédé illustré à la Figure 4), exprimée en leucocytes par millimètre carré, en fonction de la concentration mesurée WIC en leucocytes de l'échantillon E.

Dans cet exemple, la densité surfacique en leucocytes sur le plan optique PO demeure inférieure à 800 leucocytes par millimètre carré.

On a repéré trois points spécifiques sur la courbe de la Figure 6 :

- Le point A1 correspond à une concentration mesurée WIC en leucocytes égale à 10000 leucocytes par microlitre. Le taux de dilution est égal à 18. La densité surfacique de leucocytes obtenue sur le plan optique PO est égale à 570 leucocytes par millimètre carré.
- Le point A2 correspond à une concentration mesurée WIC en leucocytes égale à 50000 leucocytes par microlitre. Le taux de dilution est égal à 90. La densité surfacique de leucocytes obtenue sur le plan optique PO est égale à 750 leucocytes par millimètre carré.
- Le point A3 correspond à une concentration mesurée WIC en leucocytes égale à 200000 leucocytes par microlitre. Le taux de dilution est égal à 360. La densité surfacique de leucocytes obtenue sur le plan optique PO est égale à 570 leucocytes par millimètre carré.

Une densité surfacique cible de 570 leucocytes par millimètre carré est considérée comme très satisfaisante pour disposer de suffisamment de leucocytes sur l'image, tout en limitant leur superposition.

Le taux de dilution D est déterminé de préférence comme une fonction affine de la concentration en leucocytes de l'échantillon E initial (concentration mesurée WIC), au moins pour une partie des plages de valeurs possibles de concentration en leucocytes de l'échantillon initial, comme illustré sur la Figure 6 décrite ci-dessus. Ainsi, il est possible d'obtenir une densité surfacique très proche de 570 leucocytes par millimètre carré sur les images $I_n$, indépendamment de la concentration en leucocytes de l'échantillon E.

Le taux de recouvrement surfacique des éléments d'intérêt (ici les leucocytes) dans les images $I_n$ obtenues à l'issue de l'étape de prise de vue 500 est de préférence compris entre 0,1% et 15%, pour des leucocytes dont une dimension caractéristique moyenne est comprise entre 5 et 15 micromètres. Par « taux de recouvrement surfacique » on entend ici le nombre de pixels de l'image correspondant à des leucocytes, divisé par le nombre total de pixels de l'image.

Par exemple, le taux de recouvrement moyen des leucocytes sur les images $I_n$ est voisin de 4%, pour des leucocytes humains de dimension caractéristique moyenne égale à 9,2 micromètres. Un tel taux de recouvrement à 4% est considéré comme très satisfaisant pour disposer de suffisamment de leucocytes sur l'image tout en limitant leur superposition.

Grâce à la dilution d'ajustement à l'étape 330, la densité surfacique en leucocytes est satisfaisante, en vue des mesures sur les images issues de la prise de vue. On évite d'une part le cas représenté à gauche de la Figure 2, dans lequel le taux de recouvrement des leucocytes est insuffisant pour réaliser des mesures statistiquement pertinentes - par exemple des mesures de morphologie et de différenciation des leucocytes - et on évite d'autre part le cas représenté à droite de la Figure 2, dans lequel le nombre de leucocytes par image est trop élevé et ne permet pas de bien distinguer les caractéristiques morphologiques des leucocytes. Le nombre de leucocytes obtenu par image (ici au sein des images $I_n$), à l'issue de l'étape 500 de prise de vue, est fonction de la densité surfacique de leucocytes par millimètre carré dans le plan optique PO, et est également fonction de la surface du plan optique PO visible sur une image $I_n$.

La surface du plan optique PO visible sur une image $I_n$ est elle-même fonction du grossissement d'un capteur optique du dispositif imageur utilisé. Ici, le système de la Figure 1 comprend un dispositif photographique 5 doté d'un objectif 51 dont le grossissement est compris entre 10x et 50x. Ainsi, le nombre de leucocytes par image $I_n$ est de préférence compris entre 5 et 200, et encore plus préférentiellement entre 50 et 200. Un nombre de leucocytes par image compris entre 50 et 200 permet de réaliser un bon compromis entre, d'une part, l'évitement d'une superposition des leucocytes sur les images $I_n$ (qui nuit à la différenciation des leucocytes) et, d'autre part, l'amélioration de la précision statistique des mesures visuelles ultérieures.

Un nombre de leucocytes par image compris entre 140 et 160, par exemple égal à 150, est considéré comme optimal pour le compromis entre une faible superposition des leucocytes et une bonne précision statistique des mesures visuelles.

Un taux de recouvrement surfacique d'une image $I_n$ par des leucocytes est de préférence compris entre 0,15% et 5%. Les leucocytes ont une dimension caractéristique moyenne égale à 9,2 micromètres.

Un avantage supplémentaire de la dilution secondaire ici proposée (étape 330) est une détermination automatisée du taux de dilution D, dès lors que l'on a défini en amont certains paramètres : ici, le volume de solution qui correspond au champ visuel d'une image acquise par le dispositif photographique 5, ainsi que la concentration optimale en leucocytes qui correspond à la densité surfacique de leucocytes souhaitée sur le plan optique PO de la chambre optique 4.

A l'issue des étapes ci-avant, on dispose d'un volume de solution de test ST. Afin de réaliser la prise de vues, ce volume est transvasé dans le canal 42 de la chambre optique 4.

De façon préférentielle, comme indiqué ci-avant, on réalise ensuite une centrifugation 400 de la chambre optique 4, de sorte à ramener les leucocytes (précédemment marqués visuellement par coloration) sur un même plan optique PO de la chambre optique. L'étape de centrifugation 400 est, de préférence, réalisée automatiquement par une unité de centrifugation (non illustrée) intégrée au système de prise de vues de la Figure 1. La centrifugation est par exemple réalisée par entraînement en rotation de la chambre optique 4, autour de la direction parallèle au plan optique PO.

De manière avantageuse, le temps de centrifugation est compris entre 5 secondes et 5 minutes. De manière encore plus avantageuse, le temps de centrifugation est compris entre 10 secondes et 1 minute.

Une fois la centrifugation achevée, un nettoyage partiel est réalisé dans la chambre afin de lever les colorants en fond de vue et laisser apparaître les éléments d'intérêt fixés sur le support en verre pour que l'unité de traitement 6 commande au dispositif photographique 5 de réaliser une prise de vue de la solution ST contenue dans le canal 42.

L'image obtenue I est enregistrée dans une mémoire de l'unité de traitement.

De préférence, l'étape 500 comprend une pluralité d'acquisitions d'images $I_n$. Les moyens de déplacement sont activés pour que le dispositif photographique 5 adopte plusieurs positions successives, en se déplaçant parallèlement à l'axe de lecture A. Ainsi, le champ visuel du dispositif 5 balaie toute la longueur du canal de lecture 42.

Dans un exemple possible, environ 80 images de la solution de test ST sont acquises au total, sur une durée d'environ 30 secondes. Ainsi, le temps de centrifugation à l'étape 400 ajouté au temps de prise de vue à l'étape 500 n'excède avantageusement pas les 2 minutes.

Notamment, il n'est pas nécessaire d'attendre que les éléments d'intérêt ne sédimentent sur le plan optique PO avant de réaliser les prises de vues, grâce à la centrifugation 400. Ainsi, le temps total d'acquisition des images $I_n$ est grandement réduit, ce qui permet des mesures visuelles à la fois statistiquement pertinentes et rapides.

A une étape 600 optionnelle, une mesure visuelle est réalisée à partir de l'image I ou des images $I_n$. La mesure visuelle comporte dans le présent exemple un comptage différentiel des leucocytes, pour obtenir une répartition statistique des leucocytes de l'échantillon E (et *a fortiori* des leucocytes de l'individu) parmi les cinq sous-groupes : lymphocytes, monocytes, basophiles, éosinophiles et neutrophiles.

De façon optionnelle, la mesure visuelle réalisée à partir de l'image I ou des images $I_n$ comporte également une caractérisation qualitative des éléments d'intérêt et/ou d'autres éléments pathologiques ou non pathologiques visibles sur les images.

Comme exemple de caractérisation qualitative, une distinction peut être réalisée dans les images entre des cellules pathologiques (telles que des blastes ou des lymphomes) et des cellules non pathologiques. La détection de cellules pathologiques peut concerner les éléments d'intérêt (ici les leucocytes) et/ou d'autres éléments visibles sur les images.

La caractérisation qualitative peut notamment comporter une identification d'éléments pathologiques sur les images, comme des cellules tumorales, des parasites (malaria...) ou des bactéries caractéristiques d'un syndrome infectieux tel qu'un sepsis.

Les mesures visuelles faisant suite à la dilution d'ajustement de l'invention permettent une telle caractérisation qualitative - contrairement, par exemple, à des mesures de cytométrie de flux - tout en assurant une cadence d'analyse satisfaisante et une bonne précision statistique des mesures.

L'utilisation de telles mesures visuelles, associées à la dilution d'ajustement décrite précédemment, améliore donc la pertinence des données cliniques fournies au clinicien en sortie du procédé, tout en assurant une cadence optimale d'analyse.

La **Figure 8** compare des vues de leucocytes appartenant à cinq sous-groupes 13, 14, 15, 16 et 17 distincts, sur une prise de vue obtenue à l'issue de l'étape 500 après coloration des leucocytes. Ces sous-groupes correspondent respectivement aux éosinophiles, aux monophiles, aux neutrophiles, aux lymphocytes et aux basophiles. Le comptage différentiel des leucocytes prend en compte différentes caractéristiques morphologiques (dimensions) intéressantes des cellules : à titre d'exemples, les éosinophiles ont un noyau à plusieurs lobes et un cytoplasme présentant des granulations, les lymphocytes présentent un unique noyau occupant la majeure partie du volume cellulaire, et les neutrophiles présentent un noyau à trois lobes.

Par exemple, les résultats du comptage différentiel sont représentés graphiquement, sur une interface graphique reliée à l'unité de traitement 6, sous forme de nuage de points sur lequel on marque des clusters de points. On peut également exporter des histogrammes concernant la taille et le volume des éléments d'intérêt, la taille du noyau des éléments d'intérêt, etc.

La mesure visuelle comprend ainsi une analyse automatique des images issues de la prise de vue. De façon très avantageuse, l'analyse réalisée par ordinateur peut être vérifiée par un observateur humain. Une telle vérification est particulièrement pertinente en cas d'anomalie dans les résultats de la mesure visuelle.

En effet, contrairement à des mesures optiques telles que des mesures de diffraction, l'analyse d'images est vérifiable par un observateur. Des anomalies ou artefacts peuvent ainsi être détectés et un fonctionnement en « boîte noire » de la mesure visuelle est évité.

Selon une variante, les images acquises à l'aide de la prise de vues 500 sont enregistrées et transmises à un serveur distant qui réalise les mesures visuelles.

Un autre avantage de la mesure visuelle sur un échantillon à dilution d'ajustement est de garantir la précision statistique et la fiabilité des mesures : grâce à l'ajustement du taux de dilution, le nombre de éléments d'intérêt sur chaque vue est proche d'un nombre optimal.

Le tableau de la **Figure 9** donne le coefficient de variation statistique de la mesure de différenciation des leucocytes obtenue à l'issue de l'étape 600 pour chacun des cinq sous-groupes de leucocytes, en fonction du type de mesure de différenciation mis en oeuvre.

Les valeurs sont données pour une concentration en leucocytes WIC de l'échantillon de sang de 8000 cellules par microlitre, et pour 80 prises de vues avec un nombre cible de 150 cellules par vue. A cette concentration, les performances de la méthode à mesures visuelles sont semblables à celles de la cytométrie de flux, avec l'avantage supplémentaire d'une meilleure vérifiabilité des résultats (on évite un fonctionnement en « boîte noire »).

On a représenté en **Figure 10** l'évolution du coefficient de variation statistique des résultats de différenciation, pour le cas des lymphocytes, en fonction de la concentration en leucocytes de l'échantillon de sang. La courbe 61 correspond à une revue de lame « manuelle » par un opérateur travaillant à l'œil nu. La courbe 62 correspond à une revue de lame automatisée similaire à la méthode du document WO 2010/126903 précédemment discuté. La courbe 63 correspond à une mesure par cytométrie de flux et optique diffractive. Enfin, la courbe 64 correspond aux mesures visuelles du procédé décrit ci-avant en relation à la Figure 4.

Comme illustré sur la Figure 10, l'ajustement du taux de dilution (pour ramener la concentration en leucocytes de la solution de test ST après dilution à une concentration optimale) augmente la précision statistique par rapport aux techniques de revue de lame manuelle ou automatisée, et permet une réduction de la proportion d'échantillons qui doivent faire l'objet d'une analyse visuelle humaine. La concentration optimale en leucocytes de la solution de test ST correspond de préférence à une densité surfacique cible, notée WPD, comprise entre 20 et 1000 leucocytes par millimètre carré sur le plan optique PO de la chambre optique 4 à l'issue de la centrifugation, comme indiqué ci-avant.

De plus, le temps de recherche des éléments d'intérêt dans l'image est réduit par rapport à la technique de revue de lame automatisée, notamment dans le cas où les cellules autres que les cellules d'intérêt ont été lysées. Il n'est pas nécessaire de rechercher une zone de lecture dans un échantillon, ni d'extraire une pluralité de vues à des grossissements élevés pour réaliser les mesures morphologiques. Le procédé de l'invention permet donc également d'augmenter la cadence de traitement d'échantillons.

La **Figure 11** correspond à une vue d'ensemble des étapes de traitement d'un échantillon de sang selon un mode de réalisation préférentiel, comprenant des étapes du procédé de la Figure 4.

On utilise trois voies de mesure différentes :

- Une voie 1 comportant le comptage par impédance 150 des érythrocytes à l'aide d'une cellule disposant d'un orifice de $78\mu$m, à partir duquel on détermine une mesure de la concentration en érythrocytes et thrombocytes de l'échantillon E obtenu à l'issue du prélèvement 100 ;
- Une voie 2 comportant le comptage par impédance 200 des leucocytes à l'aide d'une cellule disposant d'un orifice de 100 $\mu$m, à partir duquel on détermine une mesure WIC de la concentration en leucocytes de l'échantillon E obtenu à l'issue du prélèvement 100 ;
- Une voie 3 comprenant, conformément au procédé décrit ci-avant en relation à la Figure 4, une détermination 300 de la nécessité d'une dilution d'ajustement, une éventuelle dilution d'ajustement 330 pour obtenir une solution de test, une centrifugation 400 de la solution de test et une prise de vues 500.

A l'étape 600, l'unité de calcul rassemble les mesures provenant des trois voies 1, 2 et 3 et effectue une série d'analyses comprenant les mesures visuelles décrites ci-avant.

Un avantage supplémentaire de la mesure visuelle est de permettre l'analyse sélective de tout type de éléments d'intérêt distinguables sur une prise de vues. La mesure visuelle est ainsi polyvalente, bien qu'elle soit de préférence appliquée ici à des leucocytes en association avec une dilution d'ajustement de la solution de test. La mesure visuelle est par exemple transposable à des érythrocytes ou à des thrombocytes, et plus généralement à tout type d'élément d'intérêt compris dans un fluide corporel.

Ainsi, si les résultats du comptage par impédance des érythrocytes sur la voie 1 sont déterminés comme anormaux, on prévoit avantageusement une « voie réflexe » de mesure consistant à réaliser un comptage des érythrocytes à l'aide de mesures visuelles. On utilise par exemple la chambre optique 4 et le dispositif photographique 5 pour réaliser une prise de vue sur laquelle les érythrocytes sont marqués.

## Revendications

1. Procédé de prise de vue d'un échantillon de fluide corporel en vue de mesures visuelles se rapportant à des leucocytes de l'échantillon, comprenant les étapes suivantes :

   - une acquisition (200) de concentration en leucocytes d'une solution issue de l'échantillon, résultant en l'obtention d'une concentration mesurée (WIC) en leucocytes de l'échantillon ;
   - une dilution (330) d'une solution de test (ST) issue de l'échantillon, selon un taux de dilution (D) déterminé en fonction de la concentration mesurée (WIC) en leucocytes de l'échantillon, de sorte à ramener la concentration en leucocytes de la solution de test (ST) à une concentration optimale de leucocytes ;
   - un transfert de la solution de test (ST) dans une chambre optique (4) et un entraînement en rotation de la chambre optique (4) par une unité de centrifugation, de sorte à réaliser un alignement des leucocytes de la solution de test (ST) sur un plan optique (PO) compris dans la chambre optique (4), le plan optique (PO) étant perpendiculaire à une direction (Z) d'épaisseur de la chambre optique (4), la concentration optimale de leucocytes pour la solution de test (ST) correspondant à une densité surfacique cible sur le plan optique (PO) comprise entre 20 leucocytes par millimètre carré et 1000 leucocytes par millimètre carré à l'issue de l'alignement des leucocytes sur le plan optique (PO),
   - une prise de vue (500), par un dispositif imageur (5), de la solution de test (ST) dans la chambre optique (4).

2. Procédé de prise de vue selon la revendication 1, dans lequel le taux de dilution D est calculé à partir de la concentration mesurée WIC en leucocytes de l'échantillon, à l'aide de la formule suivante :

$$D = WIC * h / WPD,$$

où h est la hauteur de la chambre optique (4) et où WPD est la densité surfacique cible sur le plan optique (PO).

3. Procédé de prise de vue selon l'une des revendications 1 ou 2, dans lequel le taux de dilution D est compris entre 10 et 1000.

4. Procédé de prise de vue selon l'une des revendications 1 à 3, dans lequel l'entraînement en rotation de la chambre optique (4) est réalisé pendant une durée comprise entre 5 secondes et 5 minutes, de préférence comprise entre 10 secondes et 1 minute.

5. Procédé de prise de vue selon l'une des revendications 1 à 4, dans lequel l'acquisition (200) comprend une mesure non visuelle sur une solution intermédiaire (S1) issue de l'échantillon, de préférence une mesure d'impédance par un micro-orifice et/ou une mesure d'absorbance à une longueur d'onde prédéterminée sur la solution intermédiaire issue de l'échantillon, ladite longueur d'onde étant de préférence de 540 nanomètres.

6. Procédé de prise de vue selon l'une des revendications 1 à 6, dans lequel l'étape de prise de vue (500) comprend une pluralité d'acquisitions d'images à des positions distinctes du dispositif imageur (5) par rapport à la chambre optique (4), le dispositif imageur (5) se déplaçant le long d'une même direction entre deux positions consécutives.

7. Procédé de prise de vue selon l'une des revendications 1 à 6, le procédé comprenant une étape supplémentaire de préparation de la solution de test (ST) comprenant une séparation cellulaire et/ou une lyse sélective (310) chimique et/ou physique pour réaliser un tri cellulaire conservant les leucocytes.

8. Procédé de prise de vue selon l'une des revendications 1 à 7, comprenant une étape supplémentaire de préparation de la solution de test comprenant une perméabilisation des membranes des leucocytes et/ou une coloration (320) distinctive des leucocytes.

9. Procédé de prise de vue selon l'une des revendications 1 à 8, comprenant une mesure visuelle réalisée sur l'image (I) de la solution de test, la mesure visuelle comportant de préférence un comptage différentiel (600) des leucocytes, un résultat dudit comptage comprenant une répartition des leucocytes de l'échantillon parmi une pluralité de sous-groupes.

10. Procédé de prise de vue selon l'une des revendications 1 à 9, dans lequel l'image obtenue à l'issue de la prise de vue (500) présente un nombre de leucocytes total compris entre 5 et 200, de préférence compris entre 140 et 160.

11. Système de prise de vues d'un échantillon de fluide corporel, le système étant configuré pour mettre en oeuvre un procédé de prise de vue selon l'une des revendications 1 à 10, le système comprenant :

    - un dispositif (2) de mesure, de préférence un dispositif de mesure non visuelle, de concentration en leucocytes d'une solution issue d'un échantillon de fluide corporel,

    - un processeur (6) configuré pour commander un dispositif imageur (5) et pour recevoir, depuis le dispositif (2) de mesure, une concentration mesurée (WIC) en leucocytes de l'échantillon, le processeur (6) étant en outre configuré pour calculer un taux de dilution (D) dépendant de ladite concentration mesurée (WIC),

    - un dispositif de dilution (3) adapté pour réaliser une dilution d'une solution issue d'un échantillon de fluide corporel selon un taux de dilution déterminé,
    - une chambre optique (4) adaptée pour recevoir une solution de test (ST) issue d'un échantillon de fluide corporel,
    - une unité de centrifugation configurée pour entraîner en rotation la chambre optique (4) contenant la solution de test (ST),
    - le dispositif imageur (5).

12. Système de prise de vues selon la revendication 11, dans lequel le dispositif de mesure de concentration en leucocytes comprend une cellule à micro-orifice, adaptée pour déterminer un comptage (WIC) de leucocytes par impédance.

13. Système de prise de vues selon la revendication 11 ou 12, dans lequel le dispositif imageur (5) comprend des moyens de déplacement adaptés pour déplacer un objectif (51) du dispositif imageur selon un axe de lecture (A) de la chambre optique (4).

14. Système de prise de vues selon l'une des revendications 11 à 13, dans lequel la chambre optique (4) comprend une paroi (45) de support de leucocytes délimitant un plan optique (PO), la paroi (45) de support de leucocytes présentant une épaisseur comprise entre 0,05 millimètres et 0,5 millimètres, l'unité de centrifugation étant configurée de sorte à ramener des leucocytes de la solution de test (ST) sur ledit plan optique (PO).

15. Produit programme d'ordinateur comprenant des instructions de code qui, lorsque le programme est exécuté par un processeur, conduisent le processeur à commander la mise en oeuvre d'un procédé selon la revendication 1.

**Patentansprüche**

1. Verfahren zum Bildaufnehmen einer Probe eines Körperfluids für visuelle Messungen, die sich auf Leukozyten in der Probe beziehen, das die folgenden Schritte umfasst:

   - eine Erfassung (200) der Leukozytenkonzentration in einer aus der Probe hervorgegangenen Lösung, was zum Erhalt einer gemessenen Leukozytenkonzentration (WIC) in der Probe führt;
   - eine Verdünnung (330) einer aus der Probe hervorgegangenen Testlösung (ST) gemäß einem Verdünnungsverhältnis (D), das in Abhängigkeit von der gemessenen Leukozytenkonzentration (WIC) in der Probe bestimmt wird, um die Leukozytenkonzentration der Testlösung (ST) auf eine optimale Leukozytenkonzentration zu bringen;
   - eine Übertragung der Testlösung (ST) in eine optische Kammer (4) und ein Rotationsantrieb der optischen Kammer (4) mittels einer Zentrifugiereinheit, um die Leukozyten der Testlösung (ST) in einer in der optischen Kammer (4) enthaltenen optische Ebene (PO) auszurichten, wobei die optische Ebene (PO) senkrecht zu einer Dickenrichtung (Z) der optischen Kammer (4) ist, wobei die optimale Leukozytenkonzentration für die Testlösung (ST) einer Zielflächendichte in der optischen Ebene (PO) zwischen 20 Leukozyten je Quadratmillimeter und 1000 Leukozyten je Quadratmillimeter nach Abschluss der Ausrichtung der Leukozyten in der optischen Ebene (PO) entspricht, - eine Bildaufnahme (500) der Testlösung (ST) in der optischen Kammer (4) durch eine Bildgebungsvorrichtung (5).

2. Bildaufnahmeverfahren nach Anspruch 1, wobei das Verdünnungsverhältnis D auf der Basis der gemessenen Leukozytenkonzentration WIC in der Probe mit Hilfe der folgenden Formel berechnet wird:

$$D = WIC * h / WPD,$$

wobei h die Höhe der optischen Kammer (4) ist und wobei WPD die Zielflächendichte in der optischen Ebene (PO) ist.

3. Bildaufnahmeverfahren nach einem der Ansprüche 1 oder 2, wobei das Verdünnungsverhältnis D zwischen 10 und 1000 liegt.

4. Bildaufnahmeverfahren nach einem der Ansprüche 1 bis 3, wobei der Rotationsantrieb der optischen Kammer (4) während einer Dauer zwischen 5 Sekunden und 5 Minuten, vorzugsweise zwischen 10 Sekunden und 1 Minute, durchgeführt wird.

5. Bildaufnahmeverfahren nach einem der Ansprüche 1 bis 4, wobei die Erfassung (200) eine nicht-visuelle Messung bei einer aus der Probe hervorgegangenen Zwischenlösung (S1), vorzugsweise eine Impedanzmessung durch eine Mikroöffnung und/oder eine Absorbanzmessung bei einer vorbestimmten Wellenlänge an der aus der Probe hervorgegangenen Zwischenlösung umfasst, wobei die Wellenlänge vorzugsweise 540 Nanometer beträgt.

6. Bildaufnahmeverfahren nach einem der Ansprüche 1 bis 6, wobei der Schritt der Bildaufnahme (500) eine Vielzahl von Bilderfassungen in unterschiedlichen Positionen der Bildgebungsvorrichtung (5) im Verhältnis zu der optischen Kammer (4) umfasst, wobei sich die Bildgebungsvorrichtung (5) entlang derselben Richtung zwischen zwei aufeinanderfolgenden Positionen bewegt.

7. Bildaufnahmeverfahren nach einem der Ansprüche 1 bis 6, wobei das Verfahren einen zusätzlichen Schritt der Herstellung der Testlösung (ST) umfasst, der eine chemische und/oder physikalische Zellseparation und/oder selektive Lyse (310) umfasst, um eine Zellsortierung unter Beibehaltung der Leukozyten zu erreichen.

8. Bildaufnahmeverfahren nach einem der Ansprüche 1 bis 7, umfassend einen zusätzlichen Schritt der Herstellung der Testlösung, der eine Permeabilisierung der Leukozytenmembranen und/oder eine deutliche Färbung (320) der Leukozyten umfasst.

9. Bildaufnahmeverfahren nach einem der Ansprüche 1 bis 8, umfassend eine visuelle Messung, die an dem Bild (I) der Testlösung durchgeführt wird, wobei die visuelle Messung vorzugsweise eine differentielle Zählung (600) der Leukozyten umfasst, wobei ein Ergebnis der Zählung eine Verteilung der Leukozyten der Probe auf eine Vielzahl von Untergruppen umfasst.

10. Bildaufnahmeverfahren nach einem der Ansprüche 1 bis 9, wobei das nach der Bildaufnahme (500) erhaltene Bild eine Gesamtleukozytenzahl zwischen 5 und 200, vorzugsweise zwischen 140 und 160, aufweist.

11. System zum Bildaufnehmen einer Probe eines Körperfluids, wobei das System so konfiguriert ist, dass es ein Bildaufnahmeverfahren nach einem der Ansprüche 1 bis 10 durchführt, wobei das System umfasst:

- eine Messvorrichtung (2), vorzugsweise eine nicht-visuelle Messvorrichtung, für die Leukozytenkonzentration in einer aus einer Körperfluidprobe hervorgegangenen Lösung,
- einen Prozessor (6), der so konfiguriert ist, dass er eine Bildgebungsvorrichtung (5) steuert und von der Messvorrichtung (2) eine gemessene Leukozytenkonzentration (WIC) der Probe empfängt, wobei der Prozessor (6) ferner so konfiguriert ist, dass er ein Verdünnungsverhältnis (D) berechnet, das von der gemessenen Konzentration (WIC) abhängt,
- eine Verdünnungsvorrichtung (3), die dazu geeignet ist, eine Verdünnung einer aus einer Körperfluidprobe hervorgegangenen Lösung gemäß einem bestimmten Verdünnungsverhältnis durchzuführen,
- eine optische Kammer (4), die dazu geeignet ist, eine aus einer Körperfluidprobe hervorgegangenen Testlösung (ST) aufzunehmen,
- eine Zentrifugiereinheit, die so konfiguriert ist, dass sie die optische Kammer (4), die die Testlösung (ST) enthält, in Rotation versetzt,
- die Bildgebungsvorrichtung (5).

12. Bildaufnahmesystem nach Anspruch 11, wobei die Vorrichtung zur Messung der Leukozytenkonzentration eine Zelle mit Mikroöffnung umfasst, die zur Bestimmung einer Leukozytenzählung (WIC) durch Impedanz geeignet ist.

13. Bildaufnahmesystem nach Anspruch 11 oder 12, wobei die Bildgebungsvorrichtung (5) Verlagerungsmittel umfasst, die dazu geeignet sind, ein Objektiv (51) der Bildgebungsvorrichtung entlang einer Leseachse (A) der optischen Kammer (4) zu verlagern.

14. Bildaufnahmesystem nach einem der Ansprüche 11 bis 13, wobei die optische Kammer (4) eine Leukozyten-Trägerwand (45) umfasst, die eine optische Ebene (PO) begrenzt, wobei die Leukozyten-Trägerwand (45) eine Dicke zwischen 0,05 Millimetern und 0,5 Millimetern aufweist, wobei die Zentrifugiereinheit so konfiguriert ist, dass sie Leukozyten aus der Testlösung (ST) zurück in die optische Ebene (PO) bringt.

15. Computerprogrammprodukt, umfassend Anweisungen, die, wenn das Programm von einem Prozessor ausgeführt wird, bewirken, dass der Prozessor die Durchführung eines Verfahrens nach Anspruch 1 befiehlt.

**Claims**

1. A method of imaging a sample of body fluid for visual measurements relating to leukocytes in the sample, comprising the steps of:

- acquiring (200) a leukocyte concentration in a solution obtained from the sample, resulting in a measured leukocyte concentration (WIC) in the sample;
- diluting (330) a test solution (ST) obtained from the sample according to a dilution ratio (D) which is determined as a function of the measured leukocyte concentration (WIC) in the sample in order to bring the leukocyte concentration of the test solution (ST) to an optimum leukocyte concentration;
- transferring the test solution (ST) into an optical chamber (4) and a rotational drive of the optical chamber (4) by means of a centrifuge unit in order to align the leukocytes of the test solution (ST) in an optical plane (PO) contained in the optical chamber (4), the optical plane (PO) being perpendicular to a thickness direction (Z) of the optical chamber (4), wherein the optimal leukocyte concentration for the test solution (ST) corresponds to a target area density in the optical plane (PO) between 20 leukocytes per square millimeter and 1000 leukocytes per square millimeter after completion of the alignment of the leukocytes in the optical plane (PO),
- imaging (500) the test solution (ST) in the optical chamber (4) by an imaging device (5).

2. Method according to claim 1, wherein the dilution ratio D is calculated on the basis of the measured leukocyte concentration WIC in the sample using the following formula:

$$D = WIC * h / WPD,$$

where h is the height of the optical chamber (4) and WPD is the target area density in the optical plane (PO).

3. Method according to any one of claims 1 or 2, wherein the dilution ratio D is between 10 and 1000.

4. Method according to any one of claims 1 to 3, wherein the rotation drive of the optical chamber (4) is performed for a duration between 5 seconds and 5 minutes, preferably between 10 seconds and 1 minute.

5. Method according to any one of claims 1 to 4, wherein the acquisition (200) comprises a non-visual measurement at an intermediate solution (S1) emerged from the sample, preferably an impedance measurement through a micro-aperture and/or an absorbance measurement at a predetermined wavelength at the intermediate solution emerged from the sample, the wavelength preferably being 540 nanometers.

6. Method according to any one of claims 1 to 6, wherein the imaging (500) comprises a plurality of image captures in different positions of the imaging device (5) relative to the optical chamber (4), wherein the imaging device (5) moves along the same direction between two successive positions.

7. Method according to any one of claims 1 to 6, wherein the method comprises an additional step of preparing the test solution (ST) comprising chemical and/or physical cell separation and/or selective lysis (310) to achieve cell sorting while retaining the leukocytes.

8. Method according to any one of claims 1 to 7, comprising an additional step of preparing the test solution comprising permeabilization of the leukocyte membranes and/or distinct staining (320) of the leukocytes.

9. Method according to any one of claims 1 to 8, comprising a visual measurement performed on the image (I) of the test solution, wherein the visual measurement preferably comprises a differential count (600) of the leukocytes, wherein a result of the count comprises a distribution of the leukocytes of the sample among a plurality of subgroups.

10. Method according to any one of claims 1 to 9, wherein the image obtained after the image acquisition (500) has a total leukocyte count between 5 and 200, preferably between 140 and 160.

11. System for imaging a sample of a body fluid, the system being configured to perform an imaging method according to any one of claims 1 to 10, wherein the system comprises:

   - a device (2), preferably a non-visual measuring device, for acquiring a leukocyte concentration in a solution obtained from a body fluid sample,
   - a processor (6) configured to control an imaging device (5) and to receive from the device (2) a measured leukocyte concentration (WIC) of the sample, wherein the processor (6) is further configured to calculate a dilution ratio (D) depending on the measured concentration (WIC),
   - a dilution device (3) suitable for performing a dilution of a solution obtained from a body fluid sample according to a specific dilution ratio,
   - an optical chamber (4) suitable for receiving a test solution (ST) obtained from a body fluid sample,
   - a centrifuge unit configured to rotate the optical chamber (4) containing the test solution (ST),
   - the imaging device (5).

12. System according to claim 11, wherein the device for acquiring the leukocyte concentration comprises a cell with a micro-aperture suitable for determining a leukocyte count (WIC) by impedance.

13. System according to claim 11 or 12, wherein the imaging device (5) comprises displacement means adapted to displace an objective (51) of the imaging device along a reading axis (A) of the optical chamber (4).

14. System according to any one of claims 11 to 13, wherein the optical chamber (4) comprises a leukocyte support wall (45) defining an optical plane (PO), wherein the leukocyte support wall (45) has a thickness between 0.05 millimeters and 0.5 millimeters, where the centrifuge unit is configured to return leukocytes from the test solution (ST) to the optical plane (PO).

15. A computer program product comprising instructions which, when the program is executed by a processor, cause the processor to command the execution of a method according to claim 1.

FIG. 1

# FIG. 2

## FIG. 3a

## FIG. 3b

# FIG. 4

```
                                                                    ⟋ 100
┌─────────────────────────────────────────────────────────────────┐
│              Prélèvement d'échantillon de sang                   │
└─────────────────────────────────────────────────────────────────┘
      ⟋ 200                                          ⟋ 310
       │                                              │
       ▼                                              ▼
┌──────────────────────────┐          ┌──────────────────────────────┐
│   Comptage préliminaire  │          │    Lyse des érythrocytes et  │
│ des leucocytes par impédance │      │ perméabilisation des leucocytes │
└──────────────────────────┘          └──────────────────────────────┘
   ⟋ 300         │ WIC                                 │     ⟋ 320
       │         ▼                                     ▼
┌──────────────────────────┐          ┌──────────────────────────────┐
│    Comparaison par rapport │        │         Coloration           │
│  à un seuil de concentration │      └──────────────────────────────┘
│       en leucocytes        │
└──────────────────────────┘
              │
              ▼
         ◇ Dilution ajustée nécessaire ? ◇
              │                                              ⟋ 330
              ▼                                              │
┌─────────────────────────────────────────────────────────────────┐
│          Dilution ajustée selon le taux de dilution D           │
└─────────────────────────────────────────────────────────────────┘
                              │                              ⟋ 400
                              ▼
┌─────────────────────────────────────────────────────────────────┐
│    Centrifugation de la solution ST dans la chambre optique 4   │
└─────────────────────────────────────────────────────────────────┘
                              │                              ⟋ 500
                              ▼
┌─────────────────────────────────────────────────────────────────┐
│            Prise de vues de la chambre optique 4                │
└─────────────────────────────────────────────────────────────────┘
                              │                              ⟋ 600
                              ▼
┌─────────────────────────────────────────────────────────────────┐
│            Comptage différentiel des leucocytes                 │
└─────────────────────────────────────────────────────────────────┘
```

# FIG. 5

| WIC | Méthode de dilution | Vol. solution de dilution ajouté à S2 |
|---|---|---|
| 0,3.10^3 - 7.10^3 WBC/µL | Aucune | Aucun |
| 7.10^3 - 50.10^3 WBC/µL | Ajout direct | 0 - 4,1 mL |
| 50.10^3 - 500.10^3 WBC/µL | Prélèvement de 80 µL | 0,52 - 4,92 mL |

FIG. 6

## FIG. 7

EP 3 914 893 B1

# FIG. 8

## FIG. 9

| Familles de WBC | Proportions normales dans le sang | 61 | 62 | 63 | 64 |
|---|---|---|---|---|---|
| Neutrophiles | 50% | 14.1% | 5.8% | 1.44% | 1.29% |
| Lymphocytes | 30% | 18.3% | 7.5% | 1.86% | 1.67% |
| Monocytes | 14% | 26.7% | 10.9% | 2.73% | 2.44% |
| Eosinophiles | 5% | 44.7% | 18.3% | 4.56% | 4.08% |
| Basophiles | 1% | 100.0% | 40.8% | 10.21% | 9.13% |

## FIG. 10

FIG. 11

EP 3 914 893 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2883972
- US 5812419 A
- WO 2010126903 A
- WO 2015001553 A1

**Littérature non-brevet citée dans la description**

- **MARTINAZZI MASSIMO.** A Slide Centrifugation Technic for Concentrating Blood _eukocytes and Nucleated Cells from Bone Marrow Blood. *American journal of clinical cathology,* 01 Décembre 1971